(19) Europäisches Patentamt
European Patent Office
Office européen des brevets

(11) Veröffentlichungsnummer: **0 008 077**
**B1**

(12) # EUROPÄISCHE PATENTSCHRIFT

(45) Veröffentlichungstag der Patentschrift: **16.11.83**

(21) Anmeldenummer: **79102760.0**

(22) Anmeldetag: **01.08.79**

(51) Int. Cl.³: **C 07 C 177/00,**
A 61 K 31/557,
C 07 C 35/06,
C 07 C 49/597
//C07D319/06, C07D409/14,
C07D307/935

(54) **Neue Prostaglandinderivate in der Delta-2-PGF2 und Delta-2-PGE2-Reihe, Verfahren zu deren Herstellung und ihre Verwendung als Arzneimittel.**

(30) Priorität: **04.08.78 DE 2834248**

(43) Veröffentlichungstag der Anmeldung:
**20.02.80 Patentblatt 80/4**

(45) Bekanntmachung des Hinweises auf die Patenterteilung:
**16.11.83 Patentblatt 83/46**

(84) Benannte Vertragsstaaten:
**AT BE CH DE FR GB IT NL SE**

(56) Entgegenhaltungen:
**DE - A - 2 702 553**
**DE - A - 2 753 986**
**FR - A - 2 396 749**
**FR - A - 2 403 333**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Bartmann, Wilhelm, Dr.**
**Am Dachsbau 5**
**D-6232 Bad Soden am Taunus (DE)**
Erfinder: **Beck, Gerhard, Dr.**
**Gustav-Freytag-Strasse 24**
**D-6000 Frankfurt am Main (DE)**
Erfinder: **Lerch, Ulrich, Dr.**
**Schwalbenweg 19**
**D-6238 Hofheim am Taunus (DE)**
Erfinder: **Konz, Elmar, Dr.**
**Buchenweg 22**
**D-6232 Bad Soden am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 008 077 B1**

Courier Press, Leamington Spa, England.

# Neue Prostaglandinderivate in der Δ2—$PGF_2$ und Δ2—$PGE_2$-Reihe, Verfahren zu deren Herstellung und ihre Verwendung als Arzneimittel

Prostaglandine sind eine Gruppe von Fettsäuren, die in zahlreichen Geweben und Organen von Menschen und Tier vorkommen. Das Grundgerüst der natürlichen Prostaglandine besteht aus 20 Kohlenstoffatomen, die in Form eines Fünfrings und zweier benachbarter linearer Seitenketten angeordnet sind.

Die pharmakologischen Effekte der Prostaglandine erstrecken sich u.a. auf die Gebiete der Reproduktion, des Bronchialmuskeltonus, des Blutdrucks und der Gastroenterologie. Die pharmakologischen Eigenschaften der natürlichen Prostaglandine sind Gegenstand zahlreicher Übersichtsartikel, z.B. N. H. Andersen und P. W. Ramwell in Arch. Internal Med. *133*, 30 (1974); R. L. Jones in Pathobiology Ann. *1972*, 359; J. Pike in Scient. American *225*, 84 (1971) oder M. P. L. Caton in Progress in Med. Chem. vol. 7, ed.: Butterworth, London 1971.

Die Synthesen von nicht natürlich vorkommenden Analogen von Prostansäuren, in denen die Vielzahl der pharmakologischen Wirkungen der natürlichen Prostaglandine differenziert sind, gewinnt zunehmend an Bedeutung.

Die Erfindung betrifft Verbindungen der Formel I

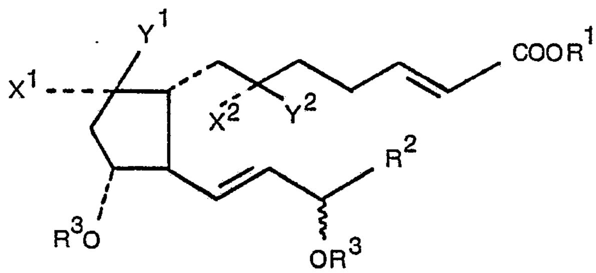

I

die strukturell mit den natürlichen Prostaglandinen verwandt sind und worin

$X^1$, $Y^1$ voneinander verschieden sind und H oder OH bedeuten,

$X^2$, $Y^2$ voneinander verschieden sind und H oder OH bedeuten, oder $X^1$ und $Y^1$ zusammen sowie $X^2$ und $Y^2$ zusammen jeweils Sauerstoff bedeuten,

$R^1$ a) Wasserstoff oder einen geradkettigen, verzweigten, gesättigten oder ungesättigten aliphatischen oder cycloaliphatischen Kohlenwasserstoff-Rest mit bis zu zehn Kohlenstoffatomen oder b) ein physiologisch verträgliches Metall-, $NH^+$-Ion oder ein Ammoniumion, das sich von einem primären, sekundären oder tertiären Amin ableitet, bedeutet,

$R^2$ einen geraden oder verzweigten Alkylrest mit eins bis sieben Kohlenstoffatomen bedeutet, in dem eine nicht endständige $CH_2$-Gruppe durch ein Sauerstoffatom ersetzt ist, oder der substituiert ist

a) mit einem α- oder β-Thienyl- oder -Furylrest, die ihrerseits im Kern 1—3 fach substituiert sein können mit Halogen, Trifluormethyl, und/oder Alkyl oder Alkoxy mit je 1—6 C-Atomen, oder

b) mit einem α- oder β-Thienyloxyrest oder einem Cycloalkoxyrest mit 3 bis 7 Kohlenstoffatomen, wobei die genannten Reste ihrerseits im Kern 1—3 fach substituiert sein können mit Halogen, Trifluormethyl und/oder Alkyl oder Alkoxy mit je 1—6 C-Atomen, und

$R^3$ Wasserstoff oder eine leicht abspaltbare Schutzgruppe, worunter z.B. eine jeweils gegebenenfalls durch mindestens ein Alkylgruppe substituierte Tetrahydropyran-2-yl- oder Tetrahydrofuran-2-yl-Gruppe oder eine 1-Äthoxyäthylgruppe zu verstehen ist, bedeutet.

Unter den Substituenten $R^1$ sind die im folgenden aufgeführten besonders bevorzugt:

Wasserstoff, Methyl, Äthyl, n-Butyl, n-Pentyl, n-Hexyl, n-Heptyl, 2-Propyl, 2-Butyl, 2-Pentyl, 3-Hexyl, 2-Methylpropyl, 2-Methylbutyl, 4,4-Dimethylpentyl, 5,5-Dimethylhexyl, Cyclopentyl, Cyclohexyl, Cycloheptyl.

Unter den Substituenten $R^2$ sind die im folgenden aufgeführten besonders bevorzugt: wie in Anspruch 1 angegeben substituierte Alkylreste oder solche, in denen eine nichtendständige $CH_2$-Gruppe durch ein Sauerstoffatom ersetzt ist, insbesondere:

1,1-Dimethyl-2-äthoxy-äthyl, 1,1-Dimethyl-2-methoxy-äthyl, 1,1-Dimethyl-cyclohexyloxymethyl, 3-Thienyl-2-äthyl, 2-Thienyl-2-äthyl, 3-(2-Chlor-thienyl)-2-äthyl, 2-(5-Chlorthienyl)-2-äthyl, 2-Thienyloxymethyl, 3-(2-Chlorthienyl)oxymethyl, 2-(5-Chlorthienyl)-oxymethyl, 3-Furyl-2-äthyl, 2-Furyl-2-äthyl.

Gegenstand der Erfindung ist ferner das in den Patentansprüchen angegebene Verfahren zur Herstellung von Verbindungen der Formel I. Die Verbindungen der Formel I können dadurch hergestellt werden,

$a_1$) ein Lactol der Formel II

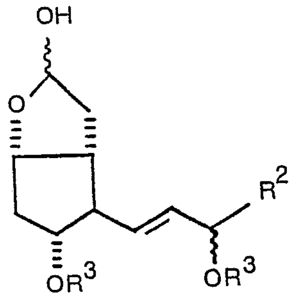

II

worin $R^2$ die zur Formel I angegebene Bedeutung hat und $R^3$ eine leicht abspaltbare Schutzgruppe bedeutet, mit einem Grignard-Reagenz der Formel III

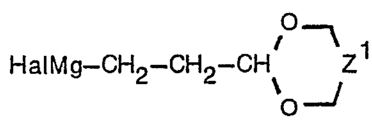

III

worin $Z^1$ eine $CH_2$-Gruppe, eine $-C(CH_3)_2$-Gruppe oder eine Einfachbindung bedeutet und Hal Chlor, Brom oder Jod bedeutet, umsetzt, zu einem Alkohol der Formel IV

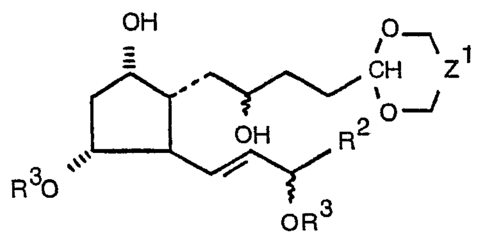

IV

worin $R^2$ und $R^3$ die zur Formel II und $Z^1$ die zur Formel III angegebene Bedeutung haben

$b_1$) in dem erhaltenen Alkohol der Formel IV durch saure Hydrolyse die Schutsgruppen abspaltet, wobei ein Aldehyd der Formel V

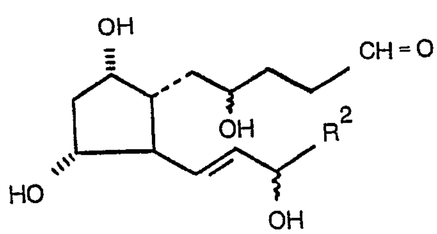

V

worin $R^2$ die in Formel I angegebene Bedeutung hat entsteht, und

$c_1$) den erhaltenen Aldehyd der Formel V umsetzt mit dem Ylid der Formel VI

$$(R^4)_3P = CH-COOCH_3 \qquad \text{VI}$$

wobei die Reste $R^4$ gleich oder verschieden sein können und geradkettiges ($C_1$—$C_4$)-Alkyl oder Phenyl bedeuten, zu einer Verbindung der Formel VII

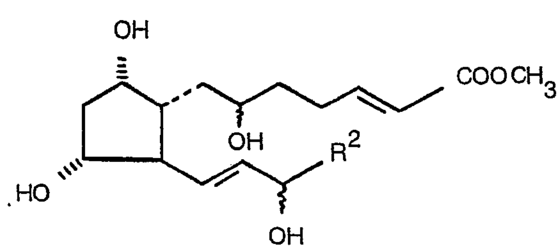

VII

worin $R^2$ die zur Formel I angegebene Bedeutung hat, oder

$d_1$) den Alkohol der Formel IV durch Oxydation in eine verbindung der Formel VIII überführt.

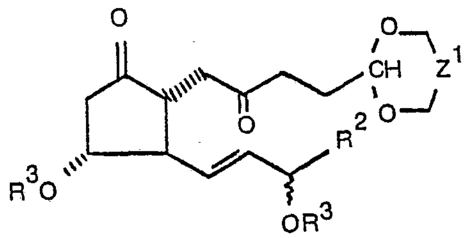

VIII

worin $R^2$, $R^3$ die zur Formel I und $Z^1$ die zur Formel III angegebene Bedeutung haben,

$e_1$) in dem Keton der Formel VIII durch saure Hydrolyse die Schutzgruppen abspaltet, wobei ein Aldehyd der Formel IX

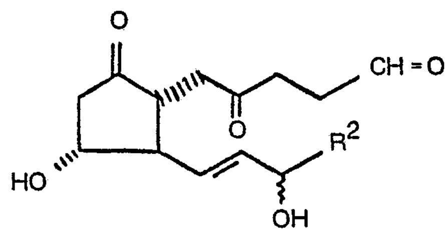

IX

worin $R^2$ die in Formel I angegebene Bedeutung hat, entsteht,

$f_1$) den Aldehyd der Formel IX umsetzt mit dem Ylid der Formel VI

$$(R^4)_3P=CH—COOCH_3 \qquad VI$$

worin die Reste $R^4$ gleich oder verschieden sein können und geradkettiges ($C_1$—$C_4$)-Alkyl oder Phenyl bedeuten, zu einer Verbindung der Formel X

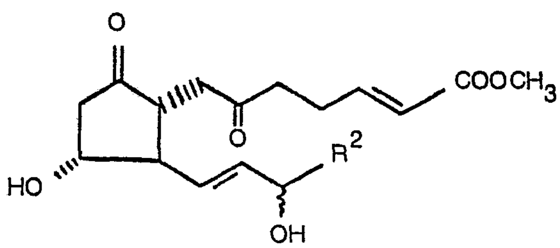

X

worin $R^2$ die in Formel I angegebene Bedeutung hat, oder

$a_2$) ein Lactol der Formel II

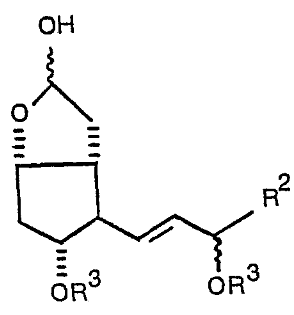

II

worin $R^2$ und $R^3$ die zur Formel I angegebene Bedeutung haben, mit einer Grignard-Verbindung der Formel XI

$$HalMg—CH_2—CH_2—CH=CH_2 \qquad XI$$

worin Hal, Chlor, Brom oder Jod bedeutet, umsetzt zu einem Alkohol der Formel XII

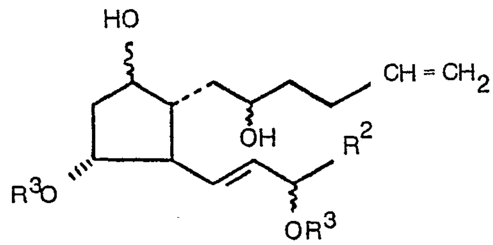

XII

worin $R^2$ und $R^3$ die zur Formel I angegebene Bedeutung haben,

$b_2$) den erhaltenen Alkohol der Formel XII oxidiert, wobei ein Keton der Formel XIII

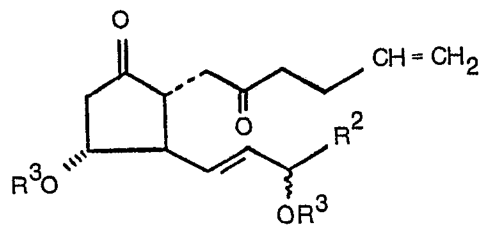

XIII

worin $R^2$ und $R^3$ die in Formel I angegebene Bedeutung haben, entsteht,

$c_2$) in dem erhaltenen Diketon der Formel XIII die endständige Doppelbindung selektiv oxidiert, wobei ein Diol der Formel XIV

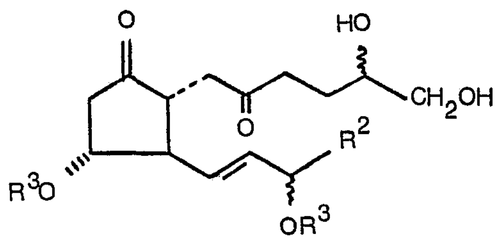

XIV

worin $R^2$ und $R^3$ die zur Formel I angegebene Bedeutung haben, entsteht,

$d_2$) das erhaltene Diol der Formel XIV oxidiert zu einem Diketoaldehyd der Formel XV

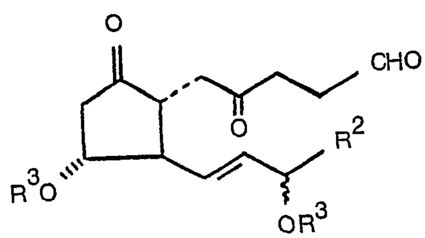

XV

worin $R^2$ und $R^3$ die in Formel I angegebene Bedeutung haben,

$e_2$) den erhaltenen Diketoaldehyd der Formel XV umsetzt mit dem Ylid der Formel VI

$$(R^4)_3P=CH—COOCH_3 \qquad VI$$

wobei die Reste $R^4$ gleich oder verschieden sind und geradkettiges ($C_1$—$C_4$)-Alkyl oder Phenyl bedeuten, zu einer Verbindung der Formel XVI

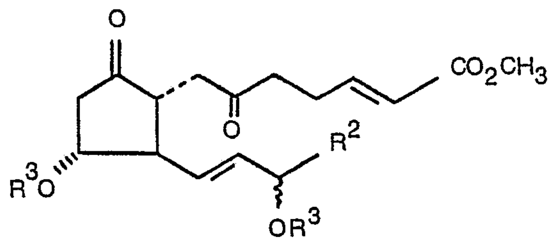

XVI

worin $R^2$ und $R^3$ die in Formel I angegebene Bedeutung haben, oder

$f_2$) in der Verbindung der Formel XV durch saure Hydrolyse die Schutzgruppen abspaltet, wobei ein Aldehyd der Formel IX

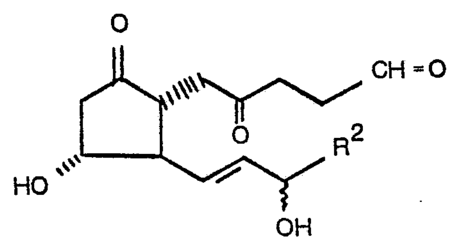

IX

worin $R^2$ die zur Formel I angegebene Bedeutung hat, entsteht, und die erhaltene Verbindung gemäss Verfahrensschritt $f_1$) mit dem Ylid der Formel VI in eine Verbindung der Formel X überführt, oder

$g_2$) gewünschtenfalls in einer Verbindung der Formel XVI

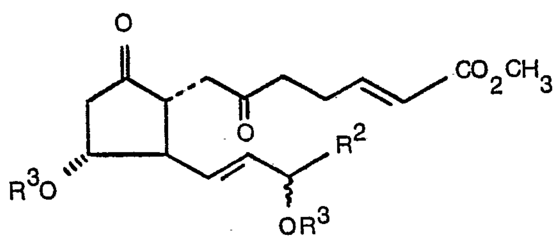

XVI

worin $R^2$ und $R^3$ die zur Formel I angegebene Bedeutung haben, durch saure Hydrolyse die Schutzgruppen $R^3$ abspaltet, wobei eine Verbindung der Formel X entsteht, und

h) gewünschtenfalls eine Verbindung der Formel X mit einem komplexen Metallhydrid reduziert zu einer Verbindung der Formel VII,

i) gewünschtenfalls eine Verbindung der Formel VII, X oder oder XVI durch Hydrolyse überführt in eine Verbindung der Formel I, worin $R^1$ Wasserstoff bedeutet und $X^1$, $Y^1$, $X^2$, $Y^2$, $R^2$ und $R^3$ die zu Formel I angegebene Bedeutung haben,

k) gewünschtenfalls eine Verbindung der Formel I, worin $R^1$ und $R^3$ Wasserstoff bedeuten, $R^2$ die zu Formel I genannte Bedeutung haben und $X^1$ und $Y^1$ sowie $X^2$ und $Y^2$ jeweils zusammen Sauerstoff bedeuten, mit einem komplexen Metallhydrid reduziert zu einer Verbindung der Formel I, worin $X^1$ und $Y^1$ sowie $X^2$ und $Y^2$ jeweils voneinander verschieden sind und Wasserstoff oder Hydroxyl bedeuten,

l) gewünschtenfalls eine Verbindung der Formel I, worin $X^1$, $X^2$, $Y^1$, $Y^2$, $R^2$ und $R^3$ die zu Formel I angegebene Bedeutung haben und $R^1$ Wasserstoff bedeutet, zu einer Verbindung der Formel I, worin $X^1$, $X^2$, $Y^1$, $X^2$, $R^2$ und $R^3$ die zu Formel I angegebenen Bedeutungen haben, wobei $R^1$ jedoch nicht Wasserstoff bedeutet, verestert,

m) gewünschtenfalls eine Verbindung der Formel I, worin $X^1$, $X^2$, $Y^1$, $Y^2$, $R^2$ und $R^3$ die zu Formel I angegebenen Bedeutungen haben und $R^1$ Wasserstoff bedeutet, in ein physiologisch verträgliches Metall- oder Aminsalz überführt.

Der Aldehyd der Formel IX bzw. XV liegt vor im Gleichgewicht mit seinem inneren Halbacetal der Formel IXa

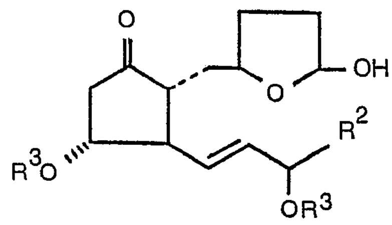

IXa

Das Verfahren zur Herstellung von Verbindungen der Formel I, worin $R^1$ Wasserstoff bedeutet, gemäss den Verfahrensschritten $a_1$) bis $f_1$) und i) kann im Einzelnen entsprechend dem in der DE—OS 25 46 313 beschriebenen Verfahren durchgeführt werden. Die Veresterung von Verbindungen der Formel I ($R^1 = H$) erfolgt nach Methoden, wie sie in der DE—OS 26 28 564 beschrieben sind.

Das Grignard-Reagenz der Formel III kann gemäss S. Büchi, H. Wüst, J. Org. Chem. 34, 1121 (1969), dargestellt werden.

Die erfindungsgemässen Verbindungen der Formel I werden gewöhnlich in Form ihrer Racemate erhalten. Diese können gegebenenfalls nach den üblichen Methoden der Racemattrennung in Form der optisch aktiven Antipoden erhalten werden.

Die Ausgangsverbindungen der Formel II können gemäss der DE—OS 24 16 193 hergestellt werden.

Das Verfahren gemäss $a_2$) bis $e_2$) geht ebenfalls aus von dem Lactol der Formel II

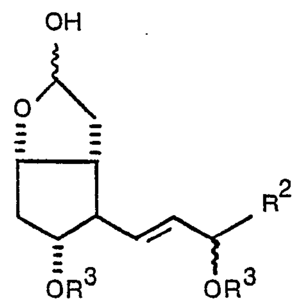

II

Dieses wird mit einer Grignard-Verbindung der Formel XI in einem aprotischen Lösungsmittel bei −20°C bis +60°C zu einem Diol der Formel XII umgesetzt, wobei eine bevorzugte Ausführungsform der Reaktion darin besteht, dass man durch Einwirkung von Magnesium auf ein 4-Halogenbuten-1,2 in Tetrahydrofuran eine Grignard-Verbindung der Formel XI darstellt und diese Grignard-Verbindung bei Raumtemperatur für 24 Stunden auf das Lactol II einwirken lässt.

Aus dem Diol der Formel XII erhält man das Diketon der Formel XIII durch Oxidation mit einem Oxidationsmittel, wie z.B. Chromsäuren, Chromsäuren-Pyridin-Komplexe, Jones-Reagenz, Pyridiniumchlorochromat, Pyridindichromat und anderen Oxidationsmitteln, die entweder Dimethylsulfoxid oder Dimethylsulfid als Reagentien benötigen. Bevorzugt wird die Oxidation in einem aprotischen Lösungsmittel bei Temperaturen zwischen −60°C und Raumtemperatur, insbesondere zwischen −60° und −5°C, in inerter Atmosphäre durchgeführt. Als Lösungsmittel kommen dabei z.B. aromatische Kohlenwasserstoffe wie Benzol oder Toluol oder z.B. chlorierte aliphatische Kohlenwasserstoffe wie Tetrachlorkohlenstoff oder Methylenchlorid in Frage.

In der Verbindung der Formel XIII wird mit einem Oxidationsmittel wie z.B. Osmiumtetroxid, Kaliumpermanganat, Persäuren die endständige Doppelbindung selektiv in einem geeigneten selbst nicht oxidierbaren Lösungsmittel wie Wasser und Dioxan bei Temperaturen von −20°C bis +50°C oxidiert, wobei ein Diol der Formel XIV entsteht, welches gegebenenfalls als solches isoliert wird oder in einem Schritt weiteroxidiert wird zu einem Aldehyd der Formel XV, gegebenenfalls unter Zusatz von Perjodsäure oder deren Alkalisalze. Im Einzelnen folgen die Oxidationsverfahren den in "Advanced Organic Chemistry" (Auflage 1976) gegebenen Vorschriften, insbesondere Kapitel B, S. 748—752 und Seiten 1087—1089.

Der erhaltene Aldehyd der Formel XV kann ohne weitere Reinigung mit einem Ylid nach Wittig zu einem Carbonsäureester der Formel XVI umgesetzt werden. Die bevorzugte Ausführungsform folgt dabei der in Fasciculus Helv. chim. acta *XL,* 1247 (1957) gegebenen Vorschrift.

Die Abspaltung der Ätherschutzgruppen in einer Verbindung der Formel XV bzw. XVI erfolgt durch schonende saure Hydrolyse durch wässrige organische Säuren, vorzugsweise in 2%iger wässrig-alkoholischer Oxalsäurelösung bei 20° bis 50°C oder durch 1- bis 2-stündiges Erhitzen in 60 bis 70%iger Essigsäure auf 40°C, wobei ein Aldehyd der Formel IX bzw. ein Ester der Formel X entsteht.

Der so erhaltene Aldehyd der Formel IX kann nach einer Filtration über eine Kieselgelsäule mit einem Ylid der Formel VI nach Wittig zu einem Carbonsäureester der Formel X umgesetzt werden,

wobei bevorzugt nach der oben angegebenen Vorschrift verfahren wird.

Die Trennung der 15—S— von dem 15—R—Epimeren erfolgt bevorzugt auf der Stufe des Esters der Formel X. Dabei erfolgt die Trennung vorzugsweise an Kieselgel (Merck® 0,063—0,20 mm) (70—230 mesh), wobei das 15—S—Epimere meist nach dem 15—R—Epimeren eluiert wird.

Der Ester der Formel X kann mit einem komplexen Metallhydrid wie etwa Lithiumaluminiumhydrid oder Natriumborhydrid oder einem anderen geeigneten Reduktionsmittel in eine Verbindung der Formel I übergeführt werden, bei der $X^1$, $Y^1$ sowie $X^2$, $Y^2$ jeweils Wasserstoff oder eine Hydroxylgruppe bedeutet, wobei $X^1$ und $Y^1$ sowie $X^2$ und $Y^2$ verschieden sind. Die bevorzugte Arbeitsweise für die Reduktion von X besteht darin, dass X in einem protischen Lösungsmittel zwischen —20°C bis Raumtemperatur mit Natriumborhydrid reduziert wird. Die bei der Reduktion anfallenden Isomeren können über eine Kieselgelsäule voneinander getrennt werden, wobei es sich als günstig erwies, über eine Merck-Fertigsäule zu chromatographieren.

Ausser den in den Beispielen genannten Verbindungen lassen sich insbesondere auch die folgenden Verbindungen nach dem erfindungsgemässen Verfahren herstellen:

6,9,11,15-Tetrahydroxy-16,16-dimethyl-18-oxa-(E)-2,(E)-13-prostadiensäure
11,15-Dihydroxy-6,9-dioxo-16,16-dimethyl-18-oxa-(E)-2,(E)-13-prostadiensäure
11,15-Dihydroxy-6,9-dioxo-16,16-dimethyl-18-oxa-(E)-2,(E)-13-prostadiensäure-äthylester
17-(2-Thienyl)-18,19,20-trinor-6-Keto-$PGE_1$
17-[3-(Chlorthienyl)]-18,19,20-trinor-6-Keto-$PGE_1$-methylester
16-[3-(2-Chlor)thienyloxy]-17,18,19,20-tetranor-6-Keto-$PGE_1$-methylester
16-(2-Thienyloxy)-17,18,19,20-tetranor-6-Keto-$PGE_1$-methylester
17-(3-Furyl)-18,19,20-trinor-6-Keto-$PGE_1$
17-Oxa-6-Keto-$PGE_1$-methylester
16,16-Dimethyl-17-oxa-21-homo-6-Keto-$PGE_1$
17-Oxa-16,16,19-trimethyl-6-Keto-$PGE_1$-n-butylester
16,16-Dimethyl-18-oxa-21-homo-6-Keto-$PGE_1$
19-Oxa-6-Keto-$PGE_1$-äthylester

Die erfindungsgemäßen Verbindungen der Formel I zeichnen sich einerseits durch spasmogene, andererseits durch Blutplättchenaggregationshemmende, ferner blutdrucksenkende, coronardilatierende und magensaftsekretionshemmende Eigenschaften aus. Verglichen mit den natürlichen Prostaglandinen sind sie stärker und länger anhaltend wirksam. Sie können daher als Arzneimittel angewandt werden.

Aus den DE—OS 27 02 553 und 27 53 986 sind 6-Keto-Verbindungen gemäß Formel I (siehe Seite 2), worin der Substituent in 15-Stellung Alkyl oder gegebenenfalls substituiertes Phenoxy darstellen kann, bekannt. Diese Verbindungen zeigen jedoch ähnlich wie die natürlichen Prostaglandine im Vergleich zu den erfindungsgemäßen Verbindungen eine wesentlich schwächere blutdrucksenkende Wirkung.

Die erfindungsgemäßen Verbindungen der Formel I können als freie Säuren, in Form ihrer physiologisch unbedenklichen anorganischen und organischen Salze oder als Ester zur Anwendung kommen.

Säuren und Salze bzw. Ester können in Form ihrer wäßrigen Lösungen oder Suspensionen oder auch gelöst oder suspendiert in pharmakologisch unbedenklichen organischen Lösungsmitteln wie ein- oder mehrwertige Alkoholen wie z.B. Äthanol, Äthylenglykol oder Glycerin, Ölen wie z.B. Sonnenblumenöl oder Lebertran, Äthern wie z.B. Diäthylenglykoldimethyläther oder auch Polyäthern wie z.B. Polyäthylenglykole der auch in Gegenwart anderer pharmakologisch unbedenklicher Polymerträger wie z.B. Polyvinylpyrrolidon zur Anwendung gelangen.

Als Zubereitungen können die üblichen galenischen Infusions- oder Injektionslösungen und Tabletten sowie örtlich anwendbare Zubereitungen wie Cremes, Emulsionen, Suppositorien, insbesondere auch Aerosole, in Frage kommen.

Die erfindungsgemässen Verbindungen der Formel I zeigen blutplättchenaggregationshemmende und blutdrucksenkende Wirkung wie durch Arachidonsäure-induzierte Bluttplättchenaggregation in vitro bzw. durch intravenöse Verabreichung am wachen Hund festgestellt, bei einer Dosis von 0,01 bis 1 mg/kg. Die Einheitsdosis zur Behandlung des Menschen liegt daher in einem Bereich von 0,01 bis 1 mg/kg, bevorzugt zwischen 0,05 und 0,5 mg/kg. Die Tagesdosis liegt zwischen 0,03 und 3 mg/kg, bevorzugt zwischen 0,15 und 1,5 mg/kg.

Die Verbindungen der Formel IV, V, VII, VIII, IX, X, XII, XIII, XIV, XV und XVI sind Zwischenprodukte für die Herstellung von Verbindungen der Formel I.

## Beispiel 1

1-Brom-2(1,3-dioxa-2-cyclohexyl)-äthan

In 851 g 1,3-Propandiol werden bei —5° 350 g Bromwasserstoff eingeleitet. Dann wurde unter Kühlung bei max. 10°C 165 g = 195 ml Acrolein zugetropft. Es wurde 1 Stunde bei R. T. nachgerührt, 4 x mit n-Hexan ausgerührt. Die organische Phase abgetrennt, zwei Stunden mit festem $NaHCO_3$

gerührt, abgesaugt, das Lösungsmittel wurde i.V. entfernt.
Rohausbeute: 408 g
Uber eine 10 cm Kolonne wurde fraktioniert.
Ausbeute: 313 g farblose Flüssigkeit Sdp $_{15\ mm}$ = 89—91 °C

NMR: δ1, 1—2, 4 (m, 4) $—CH_2—$, $—CH_2—$, $—CH_2—CH<$;
δ3, 3—4, 3 (m, 6) $—CH_2—Br$, $—CH_2—O—$
δ4, 6 (t, 1) —O—CH—O—

Beispiel 2a)

2-Oxa-3-hydroxy-6(3-tetrahydropyranyloxy-1-octenyl)-7-tetrahydropyranyloxy-bicyclo[3,3,0]octan II

10,32 g (0,025 Mol) 2-Oxa-3-oxy-6(3-tetrahydropyranyloxy-1-octenyl)-7-tetrahydropyranyloxy-bicyclo[3,3,0]-octan dargestellt analog DE—OS 2,416,193 werden in 250 ml Toluol abs. aufgenommen und auf —70°C abgekühlt. Dann werden 29,25 ml 20%ige Diisobutylaluminiumhydridlösung in abs. Toluol innerhalb 1 Stunde zugetropft. Anschließend wird 2 Stunden nachgerührt bei —70°C.

Dann werden 2,5 ml Essigsäure in Toluol zugetropft. Man läßt auf 0°C kommen und versetzt mit 100 ml gesättigter Natriumchloridlösung. Man saugt über eine Klarschicht ab. Das Filtrat wird mit Äther-$H_2O$ versetzt. Die organische Phase wird abgetrennt, getrocknet und i.Vak. eingeengt.
Ausbeute: 9,4 g farbloses Öl
Dünnschichtchromatogramm:
$R_f$ = 0,06 Benzol/Äthylacetat 4:1
IR-Spektrum: (NaCl-Platten)
3400 (OH-Bande), 2930 *keine* Carbonylbande,
1450, 1200, 1122, 1035 und 980 $cm^{-1}$

Beispiele 2 b) bis 2 f)

In Analogie zu Beispiel 2 a) lassen sich die folgenden Verbindungen durch Reduktion mit Diisobutylaluminiumhydrid herstellen:

| Beispiel 2 | $R^2$ | $R_f$-Werte in Essigester |
|---|---|---|
| b) | 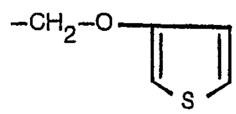 | 0.82 |
| c) | 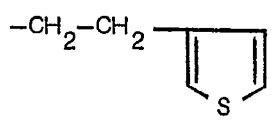 | 0.78 |
| d) | 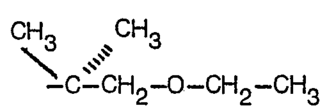 | 0.53 (Essigester 2/ Cyclohexan 1) |
| e) | 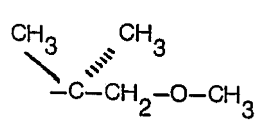 | 0.92 |
| f) | 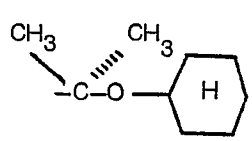 | 0.91 |

Beispiel 3

1[4(3-Hydroxy-1(1,3-dioxa-2-cyclohexyl-)butyl]-2(3-tetrahydro-pyranyloxy-1-octenyl)-3-(tetrahydropyranyloxy)-5-hydroxy-cyclopentan IV

170 mg Magnesium werden mit Jodkristallen angeätzt, mit 5 ml abs. THF versetzt und 1 g 1 - Brom - 2(1,3 - dioxa - 2 - cyclohexyl) - äthan (Beispiel 1) in 5 ml abs. THF innerhalb 20 Minuten zugetropft, (Reaktion springt beim leichten Erwärmen an) dann wird 2 Stunden bei 50°C weitergerührt und 440 mg 2 - Oxa - 3 - hydroxy - 6(3 - tetrahydropyranyloxy - 1 - octenyl) - 7 - tetrahydropyranyloxy - bicyclo[3,3,0] - octan (Beispiel 2a) in 10 ml abs. Diäthyläther zugetropft. Man rührt 3 Stunden bei Raumtemperatur weiter.

Es wird mit Eis und verdünnter Salzsäure versetzt, dann mit NaCl gesättigt und mit Äther extrahiert. Die organische Phase wird 2 x mit NaCl/Bicarbonat-Lösung gewaschen mit $MgSO_4$ getrocknet, i. Vak. eingeengt.

Rohausbeute: 610 mg

Über eine Kieselgelsäule mit Äther als Elutionsmittel wurden Verunreinigungen abgetrennt.

Ausbeute: 400 mg leicht gelbes Öl

$R_f$ = 0,06 Cyclohexan 50/Essigester 50/Eisessig 2

NMR:$\delta$ 0, 7—2, 5 (m, 35) —$CH_2$—, —CH—;
3, 1—4, 3 (m, 14) —$CH_2$—O—, —CH—O—, OH;
4, 35—4, 8 (m, 3) —O—CH—O,
5, 2—5, 6 (m, 2) olefinin. Protonen

Beispiel 4

1[4(1-Formyl-3-hydroxy)-butyl]-2(3-hydroxy-1-octenyl)-3,5-dihydroxy-cyclopentan V

390 mg 1[4(3-Hydroxy-1(1,3-dioxa-2-cyclohexyl)-butyl]-2-(3-tetrahydropyranyloxy-1-octenyl)-3-(tetrahydropyranyloxy)-5-hydroxy-cylopentan (Beispiel 3), 5 ml Dimethylglykol 5 ml Wasser und 1 ml gesättigter Oxalsäurelösung werden 13 Stunden bei 50°C gerührt. Es wird abgekühlt, mit 10 ml gesättigter Natriumchlorid-Lösung versetzt, mit NaCl gesättigt, mit Essigester extrahiert. Die organische Phase wird 1 x mit gesattigter NaCl-Lösung/Bicarbonatlösung gewaschen über $MgSO_4$ getrocknet, i. Vak. eingeengt.

Über eine Säule aus Kieselgel mit anfangs Äther/Essigester 1:1 als Elutionsmittel später mit Essigester/Eisessig 99:1 wird von Verunreinigungen abgetrennt.

Ausbeute: 200 mg leicht gelbes Öl *2 Isomere*

$R_f$Wert = 0,19 Essigester: Eisessig = 9:1 0,13

NMR: $\delta$ 0, 8—2, 7 (m, 21) —$CH_2$—, —CH—;
3, 5—4, 6 (m, 7) —CH—O—, OH;
5, 4—3, 7 (m, 2) olefin. Protonen

Beispiel 5

6,9,11,15-Tetrahydroxy-(E)-2, (E)-13-prostadiensäure-methylester VII

190 mg 1[4(1-Formul-3-hydroxy)-butyl]-2(3-hydroxy-1-octenyl)-3,5-dihydroxy-cyclopentan V (Beispiel 4) sowie 290 mg Carbomethoxymethylen-triphenyl-phosphoran VI (Lit: v. Fasciculus Helv. chim. acta *XL*, S. 1247 (1957)) werden in 10 ml abs. Benzol unter $N_2$ 16 Stunden bei Raumtemperatur gerührt. Es wird eingeengt, der Rückstand über eine Säule aus Kieselgel gereinigt. Elutionsmittel 1. Äther, 2. Äther/Essigester und 3. Essigester/Essigsäuremethylester.

Ausbeute: 43 mg hellgelbes Öl *2 Isomere*

$R_f$Werte 0,26 Essigester: Eisessig = 9:1 0,22

NMR: $\delta$ 0, 8—2, 5 (m, 21) —$CH_2$—, —CH—,
2, 6—3, 2 (m, 4) OH
3, 5—4, 4 (m, 4) —*C*H—OH,
3, 7 (s, 3) *O*$CH_3$,
5, 4—6, 1 (m, 2) olefin. Prot.,
6, 6—7, 1 (m, 2) olefin. Prot,

Beispiel 6 a)

6,9,11,15-Tetrahydroxy-(E)-2, (E)-13-prostadiensäure I

300 mg 6,9,11,15-Tetrahydroxy-(E)-2, (E)-13-prostadiensäuremethylester VII (Beispiel 5) werden in 10 ml Methanol gelöst und mit 3 Äquivalenten 0,5 N Natronlauge versetzt. Man rührt bei Raumtemperatur 8 Stunden und verfolgt den Reaktionsverlauf im Dünnschichtchromatogramm. Nach Beendigung der Verseifung wird das Lösungsmittel vorsichtig im Vakuum entfernt, der Rückstand mit Essigester/Wasser versetzt, mit 1 N Citronensäure auf pH 4—5 angesäuert. Die org. Phase abgetrennt, mit $MgSO_4$ getrocknet, i.Vak. eingeengt.

Ausbeute: 270 mg gelbbraunes Öl, welches durch Chromatographie an einer Kieselgelsäule mit Essigsäureäthylester/Eisessig 95: 5 gereinigt wird.

Reinausbeute: 160 mg hellgelbes Öl

NMR: $\delta$ 0,8—2,5 (m, 21) —$CH_2$—, —CH—,
3,5—4,3 (m, 4) —*CH*—OH,
4,5—5,3 (breites Signal, 5) O*H*, COO*H*
5,4—6,1 (m, 2) olefin. Protonen,
6,6—7,1 (m, 2) olefin. Protonen

Beispiele 6 b) bis 6 f)

In Analogie zu Beispiel 6 a) lassen sich aus den Verbindungen der Beispiele 22 b) bis 22 f) durch Esterverseifung die folgenden Verbindungen herstellen:
(Formel I, $X^1$, $Y^1$ und $X^2$, $Y^2$ = H bzw. OH, $R^1$ = H)

| Beispiel 6 | $R^2$ |
|---|---|
| b) | 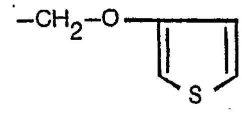 |
| c) | 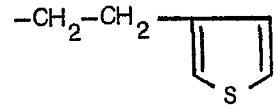 |
| d) | 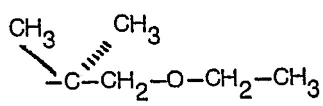 |
| e) | 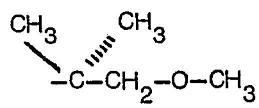 |
| f) | 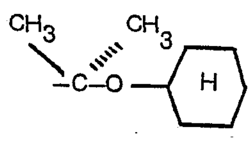 |

Beispiel 7

1[4(3-Oxo-1(1,3-dioxa-2-cyclohexyl)-butyl]-2(3-tetrahydropyranyloxy-1-octenyl)-3(tetrahydropyranyloxy)-5-oxo-cyclopentan VIII

0,45 g 1[4(3 - Hydroxy - 1(1,3 - dioxa - 2 - cyclohexyl) - butyl] - 2(3 - tetrahydro - pyranyloxy - 1 - octenyl) - 3(tetrahydropyranyloxy) - 5 - hydroxy - cyclopentan IV (Beispiel 3) werden in 30 ml Aceton gelöst. Bei —20 bis —25°C werden unter Argon 2 ml Iones Reagenz (2,1 g Chromsäure, 6 ml Wasser, 1,7 ml konz. Schwefelsäure) zugetropft. Man rührt 30 Minuten, gibt dann 3 ml Isopropanol zu und rührt weitere 10 Minuten, um überschüssiges Oxydationsreagenz zu zerstören. Es wurde dann mit 100 ml Methylenchlorid und 100 ml Wasser versetzt, ausgeschüttet, die Phasen getrennt, der organische Extrakt mit $MgSO_4$ getrocknet und das Lösungsmittel im Vakuum bei maximal +5°C eingeengt. Die Ausbeute an der Verbindung VIII betrug 0,32 g fast farbloses, klares Öl. Dünnschichtchromatogramm (Essigsäureäthylester — Essigsäure = 97,5:2,5) auf Kieselgelplatten-Merck

$R_f$ = 0,75

Beispiel 8

1[4(1-Formyl-3-oxo)-butyl]-2(3-hydroxy-1-octenyl)-3-hydroxy-cyclopentan-5-on IX

280 mg VIII von Beispiel 7, 5 ml Dimethylglykol, 5 ml Wasser und 1 ml gesättigte Oxalsäurelösung werden 13 Stunden bei 50°C gerührt. Es wird abgekühlt, mit 10 ml gesättigter Natriumchlorid-Lösung versetzt, mit NaCl gesättigt, mit Essigester extrahiert. Die org. Phase wird 1 x mit ges. NaCl-Lösung/Bicarbonatlösung gewaschen über $MgSO_4$ getrocknet, i. Vak. eingeengt. Über eine Säule aus Kieselgel mit anfangs Äther/Essigester 1:1 als Elutionsmittel später mit Essigester/Eisessig 99:1 wird von Verunreinigungen abgetrennt.

Ausbeute: 170 mg farbloses Öl
Dünnschichtchromatogramm (Essigsäureäthylester — Eisessig = 97,5:2,5) auf Kieselgelplatten-Merck
$R_f = 0,55$

Beispiel 9 a)

6,9-Dioxo-11,15-dihydroxy-(E)-2, (E)-13-prostadiensäuremethylester X

170 mg 1[4(1-Formyl-3-oxo-)butyl]-2(3-hydroxy-1-octenyl)-3-hydroxy-cyclopentan-5-on IX (Beispiel 8) sowie 240 mg Carbomethoxymethylen-triphenyl-phosphoran VI (Lit: v. Fasciculus Helv. chim.acta *XL*, S. 1247 (1957)) werden in 15 ml abs. Benzol unter $N_2$ 16 Stunden bei Raumtemperatur gerührt. Es wird eingeengt, der Rückstand über eine Säule aus Kieselgel gereinigt. Elutionsmittel 1. Äther, 2. Äther/Essigester und 3. Essigester/Essigsäuremethylester.
Ausbeute: 123 mg hellgelbes Öl *2 Isomere*
Dünnschichtchromatogramm: Kieselgelplatten der Fa. Merck

$R_f$ Werte: 0,5 (Essigester:Eisessig = 97,5:2,5)
0,45

NMR: $\delta$ 0,8—2,5 (m, 21) —$CH_2$—, —CH—,
3,0—3,5 (m, 2) OH
3,5—4,4 (m, 2) —*CH*—OH,
3,8 (s, 3) *$OCH_3$*,
5,5—6,2 (m, 2) olefin. Prot.,
6,6—7,2 (m, 2) olefin. Prot.

Beispiele 9 b) bis 9 f)

In Analogie zu Beispiel 9 a) lassen sich aus den Verbindungen 20 b) bis 20 f) durch Wittig-Reaktion die Verbindungen 9 b) bis 9 f) darstellen:
(Formel X)

| Beispiel 9 | $R^2$ | δ-Werte NMR ($CDCl_3$) |
|---|---|---|
| b) | 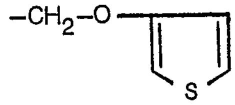 | 0.95—3.0 (m, 12H) $—CH_2—$, $—CH—$, OH; 3.6 (s, H) $—OCH_3$; 3.9 (d, 2H) $—CH_2—O—$ Thiophen; 4.1—4.6 (m, 2H) $—CH—OH$; 5.4—5.8 (m, 2H) olefin. Prot.; 6.0—7.3 (m, 5H) Thiophen und olefin. Prot. |
| c) | 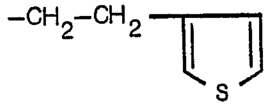 | 0.95—3.0 (m, 16H) $—CH_2—$, $—CH—$, OH; 3.6 (s, 3H) $—OCH_3$; 3.7—4.5 (m, 2H) $—CH—OH$; 5.3—5.9 (m, 2H) olefin. Prot.; 6.0—7.3 (m, 5H) olefin. Prot. und Thiophen. |
| d) | 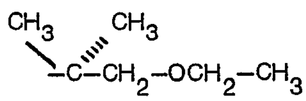 | 0.9 (s, 6H) $C(CH_3)_2$; 1.15 (t, 3H) $—CH_2—CH_3$; 1.4—2.95 (m, 16H) $—CH_2—$; $—CH—$, OH; 3.3 (s, 2H) $—OCH_2—$; 3.5 (q, 2H) $—OCH_2CH_3$; 3.6 (s, 3H) $OCH_3$; 3.8—4.5 (m, 2H) $—CH—OH$; 5.5—7.1 (m, 4H) olefin. Prot. |
| e) | 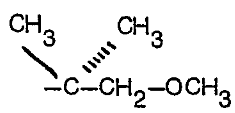 | 0.9 (s, 6H) $C(CH_3)_2$; 1.1—2.9 (m, 12H) $—CH_2—$; $—CH—$, OH; 3.2. (s, 2H) $—CH_2OCH_3$; 3.3 (s, 3H) $—CH_2—OCH_3$; 3.6 (s, 3H) $—CO_2CH_3$; 3.7—4.5 (m, 2H) $—CH—OH$; 5.5—7.1 (m, 4H) olefin. Prot. |
| f | 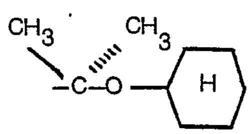 | 1.05 (d, 6H) $—C(CH_3)_2$; 0.9—3.1 (m, 19H) $—CH_2—$, $—CH—$, OH; 3.6 (s, 3H) $—CO_2CH_3$; 3.4—4.3 (m, 3H) $—CH—OH$, $—CH—O$; 5.5—7.1 (m, 4H) olefin. Prot. |

Beispiel 10 a)

5,9-Dioxo-11,15-dihydroxy-(E)-2, (E)-13-prostadiensäure I

123 mg 6,9-Dioxo-11,15-dihydroxy-(E)-2, (E)-13-prostadiensäure-methylester X (Beispiel 9 a) werden in 15 ml Methanol gelöst und mit 4 Äquivalenten 0,5 N Natronlauge versetzt Man rührt bei Raumtemperatur 8 Stunden und verfolgt den Reaktionsverlauf im Dünnschichtchromatogramm. Nach Beendigung der Verseifung wird das Lösungsmittel vorsichtig im Vakuum entfernt, der Rückstand mit Essigester/Wasser versetzt, mit 1 N Citronensäure auf pH 4—5 angesäuert. Die org. Phase abgetrennt mit $MgSO_4$ getrocknet, i. Vak. eingeengt.

Ausbeute: 130 mg gelbbraunes Öl, welches durch Chromatographie an einer Kieselgelsäule mit Essigsäureäthylester/Eisessig 95:5 gereinigt wird.

Reinausbeute: 86,0 mg helles Öl
NMR: $\delta$ 0,8—2,5 (m, 21) —*CH*—, —CH—,
3,5—4,3 (m, 2) —*CH*—OH,
4,8—5,4 (breites Signal, 3) O*H*, COO*H*
5,4—6,2 (m, 2) olefin. Protonen
6,5—7,1 (m, 2) olefin. Protonen

Beispiele 10 b) bis 10 f)

In Analogie zu Beispiel 10 a) lassen sich aus den Verbindungen 9 b) bis 9 f) durch Esterverseifung die Verbindungen 10 b) bis 10 f) darstellen:
(Formel I, $X^1 = Y^1 = X^2 = Y^2 = O$)

| Beispiel 10 | $R^2$ |
|---|---|
| b) | 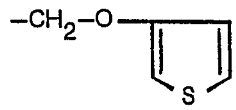 |
| c) | 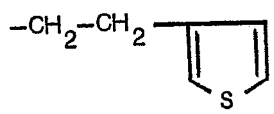 |
| d) | 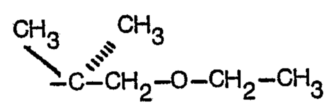 |
| e) | 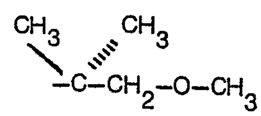 |
| f) | 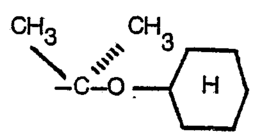 |

Beispiel 11 a)

6,9,11,15-Tetrahydroxy-(E)-2, (E)-13-prostadiensäureäthylester I

Eine Lösung von 80 mg 6,9,11,15-Tetrahydroxy-(E)-2, (E)-13-prostadiensäure I (Beispiel 6a) in 6 ml Äther wird unter Eiskühlung mit 5 ml einer 1 M Diazoäthanlösung in Äther versetzt. Man lässt noch 30 Minuten rühren und verdampft das Lösungsmittel mit dem überschüssigem Diazoäthan im Vakuum. Das Produkt ist chromatographisch rein.
NMR: analog Beispiel 6,
Ester: $\delta$ 1,25 (t, 3) —$COOCH_2CH_3$;
4,25 (q, 2) —$COOCH_2CH_3$

Beispiele 11 b) bis 11 f)

In Analogie zu Beispiel 11 a) lassen sich aus den Verbindungen der Beispiele 6 b) bis 6 f) die entsprechenden Äthylester darstellen.

Beispiel 12 a)

6,9-Dioxo-11,15-dihydroxy-(E)-2, (E)-13-prostadiensäureäthylester I

Eine Lösung von 50 mg 6,9-Dioxo-11,15-dihydroxy-(E)-2, (E)-13-prostadiensäure (Beispiel 10 a) in 10 ml Äther wird unter Eiskühlung mit 5 ml einer 1 M Diazoäthanlösung in Äther versetzt. Man lässt noch 30 Minuten rühren und verdampft das Lösungsmittel mit dem überschüssigen Diazoäthan im Vakuum. Das Produkt ist chromatographisch rein.
NMR: analog Beispiel 10a
Ester: $\delta$ 1,24 (t, 3) —$COOCH_2CH_3$,
4,25 (q, 2) —$COOCH_2CH_3$

Beispiele 12 b) bis 12 f)

In Analogie zu Beispiel 12 a) lassen sich aus den Verbindungen der Beispiele 10 b) bis 10 f) die entsprechenden Äthylester darstellen.

Beispiel 13 a)

6,9,11,15-Tetrahydroxy-(E)-2, (E)-13-prostadiensäure-Thromethaminsalz I

Eine Lösung von 70 mg 6,9,11,15-Tetrahydroxy-(E)-2, (E)-13-prostadiensäure (Beispiel 6a) in 4 ml Äthanol wird mit einer äthanolischen Lösung von 22,9 mg Thromethaminbase versetzt und das Lösungsmittel abgedampft, zuletzt im Hochvakuum.

Ausbeute: 92 mg Thromethaminsalze I ("ölige Kristalle")

Beispiele 13 b) bis 13 f)

In Analogie zu Beispiel 13 a) lassen sich aus den Verbindungen der Beispiele 6 b (bis 6 f) die entsprechenden Salze herstellen.

Beispiel 14 a)

6,9-Dioxo-11,15-dihydroxy-(E)-2, (E)-13-prostadiensäure-Thromethaminsalze I

Eine Lösung von 46 mg 6,9-Dioxo-11,15-dihydroxy-(E)-2, (E)-13-prostadiensäure (Beispiel 10 a) in 3 ml Äthanol wird mit einer äthanolischen Lösung von 14, 9 mg Thromethaminbase versetzt und das Lösungsmittel abgedampft, zuletzt im Hochvakuum.

Ausbeute: 59,5 mg Thromethaminsalz I

Beispiel 14 b bis 14 f

In Analogie zu Beispiel 14 a lassen sich aus den Verbindungen der Beispiele 10 b bis 10 f) die entsprechenden Salze herstellen.

Beispiel 15 a)

1 - [6(5 - Hydroxy) - hex - 1 - en - yl] - 2(3 - tetrahydro - pyranyloxy - 1 - octenyl] - 3 - (tetrahydro-pyranyloxy) - 5 - hydroxy - cyclopentan (XII). 250 mg Magnesium werden mit 0,2 ml 1,2-Dibromäthan angeätzt, mit 5 ml abs. THF versetzt und 1,1 g 4-Brombuten-1,2 in 5 ml abs. THF innerhalb 20 Minuten zugetropft (Reaktion springt beim leichten Erwärmen an), dann wird 3 h bei Raumtemperatur weitergerührt und 0,88 g 2 - Oxa - 3 - hydroxy - 6(3 - tetrahydropyranyloxy - 1 - octenyl) - 7 - tetrahydropyranyloxybicyclo[3,3,0] - octan (Beispiel 2 a) in 10 ml abs. Diäthyläther zugetropft. Man rührt 24 Stunden bei Raumtemperatur weiter.

Es wird mit Eis und verdünnter Salzsäure versetzt, dann mit NaCl gesättigt und mit Äther extrahiert. Die organische Phase wird 2 x mit NaCl/Bicarbonat-Lösung gewaschen, mit $MgSO_4$ getrocknet, i. Vak. eingeengt.

Rohansbeute: 1 g

Das Rohprodukt wurde ohne weitere Säuberung für den nächsten Schritt eingesetzt.

$R_f = 0,13$ Benzol/Äthylacetat 1:1

NMR: $\delta$ 5,2—5,8 (m, 2H) olefin. Prot.

5,2—4,5 (3m, 5H) olefin. Prot., 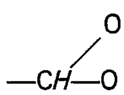

4,5—3,0 (m, 8H) OH; —$CH$—O

2,7—0,8 (m, 33) —$CH_2$—; —$CH$—

Beispiel 15 b bis 15 f

In Analogie zu Beispiel 15 a lassen sich aus den Verbindungen der Beispiele 2 b bis 2 f durch Grignard-Reaktion mit einem 4-Halogen-buten-1,2 die folgenden Verbindungen darstellen (Formel XII):

| Beispiel 15 | $R^2$ | δ-Werte NMR ($CDCl_3$) |
|---|---|---|
| b) | 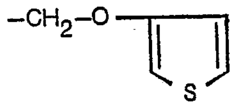 | 3,9 (d, 2H) —$CH_2$—O-Thiophen 4, 1—4,6 (m, 2H); 4,6—5,2 (3m, 5H) olefin. Prot; 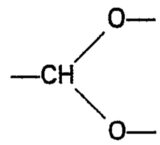 5,2—5,8 (m, 2H) olefin. Prot., 6,1—7,3 (m, 3H) Thiophen |
| c) | 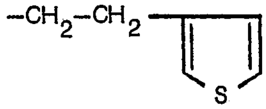 | 4,6—5,2 (3m, 5H) olefin. Prot. 5,4—5,8 (m, 2H) olefin. Prot., 6,8—7,3 (m, 3H) Thiphen |
| d) | 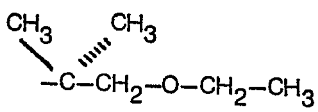 | 0,9 (s, 6H) $C(CH_3)_2$; 1,15 (t, 3H) —$CH_2$—$CH_3$ 4,6—5,2 (3m, 5H) und 5,4—5,8 (m, 2H) olefin. Protonen und 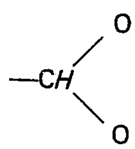 |
| e) | 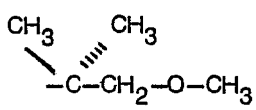 | 0,9 (s, 6H) —C—$(CH_3)_2$; 3,2 (s, 2H) —$CH_2$—$OCH_3$; 3,3 (s, 3H —$CH_2$—$OCH_3$ 4,6—5.8 (4m, 7) olefin. Prot. und 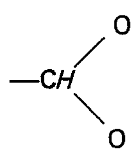 |
| f) | 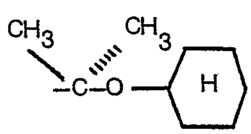 | 1.05 (d, 6H) —$(CCH_3)_2$; 4.5—5,2 (3m, 5H), 5.4—5.8 (m, 2H) olefin. Prot. und 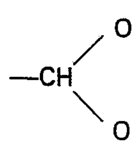 |

### Beispiel 16 a

1[6(5-oxo-)-hex-1-en-yl]-2[3-tetrahydropyranyloxy-1-octenyl]-3-(tetrahydro-pyranyloxy)-5-oxo-cyclopentan (XIII)

2.1 ml DMSO werden bei −60°C zu 20 ml $CH_2Cl_2$ zugetropft und anschließend werden 2.8 ml Trifluoressigsäureanhydrid in 10 ml $CH_2Cl_2$ bei gleicher Temperatur zugefügt. Es wird 20 Minuten nachgerührt, anschließend 1.0 g 1[6(5 - Hydroxy) - hex - 1 - en - yl] - 2(3 - tetrahydro - pyranyloxy - 1 - octenyl] - 3 - (tetrahydro - pyranyloxy) - 5 - hydroxy - cyclopentan (XII) in 10 ml $CH_2Cl_2$ bei −70°C zugetropft, 20 Minuten nachgerührt und mit 10 ml Triäthylamin versetzt, weitere 30 Minuten bei −60°C gerührt und dann langsam auf Raumtemperatur erwärmt. Es wird mit 30 ml gesättigter Natriumchlorid-Lösung versetzt, mit Essigester extrahiert. Die organische Phase wird noch 1 × mit ges. NaCl-Lsg/Bicarbonatlösung gewaschen, über $MgSO_4$ getrocknet, i. Vak. eingeengt.

Über eine Kieselgelsäule wird mit Cyclohexan:Essigester 2:1 von Verunreinigungen abgetrennt.
Ausbeute 855 mg Öl

NMR: 5.8—4,4 (4m, 7H)
4.2—3.0 (m, 7H)
3—2 (m, 9H)
2—0.8 (m und 1t, 22H)

### Beispiel 16 b bis 16 f

In Analogie zu Beispiel 16 a lassen sich aus den Verbindungen der Beispiele 15 b bis 15 f durch Oxidation die folgenden Verbindungen darstellen, (Formel XIII):

| Beispiel 16 | $R^2$ | $R_f$-Werte (in Essigester) |
|---|---|---|
| b | 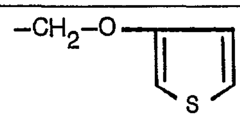 | 0.63 |
| c | 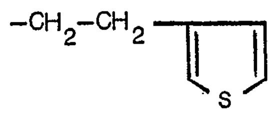 | 0.57 |
| d | 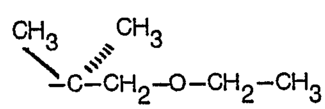 | 0.80 |
| e | 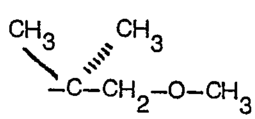 | 0.68 |
| f | 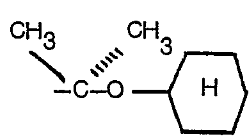 | 0.070 |

### Beispiel 17a

1[6(1,2-Dihydroxy-5-oxo-)-hexyl]-2[3-tetrahydro-pyranyloxy-1-octenyl]-3-[tetrahydro-pyranyloxy)-5-oxo-cyclopentan (XIV)

78.5 mg 1[6(5 - oxo) - hex - 1 - en - yl] - 2[3 - tetrahydro-pyranyloxy - 1 - octenyl] - 3 - (tetrahydro - pyranyloxy) - 5 - oxo - cyclopentan (XIII) werden in 3 ml abs. Pyridin gelöst und bei 0°C mit 41 mg Osmium tetroxid versetzt und 4 Stunden bei 0°C gerührt. Nach dieser Zeit wird 90 mg $NaHSO_3$ in einem ml Wasser hinzugetropft und eine Stunde bei Raumtemperatur gerührt. Man versetzt mit 10 ml ges. NaCl-Lsg. und extrahiert, die organische Phase wird mit ges. NaCl-Lsg. gewaschen, über $MgSO_4$ getrocknet und i. Vak. eingeengt und das Rohprodukt noch 3 × mit Toluol abrotiert.

Über eine Kieselgelsäule wird mit Cyclohexan/Essigester 1:1 und anschließend Essigester nicht umgesetztes Ausgangsmaterial vom Produkt abgetrennt.

41 mg Ausgangsmaterial.
Ausbeute: 32 mg
$R_f$-Wert = 0.2 (Essigester)
NMR: 5.5 (m, 2H)
4.5 (m, 2H)
4.4—3 (m, 10H)
2.9—0.8 (m und 1t, 34H)

Beispiel 17 b bis 17 f

In Analogie zu Beispiel 17 a lassen sich aus den Verbindungen der Beispiele 16 b bis 16 f durch Oxidation die folgenden Diole herstellen. (Formel XIV)

| Beispiel 17 | $R^2$ | $R_f$-Wert (in Essigester) |
|---|---|---|
| b | 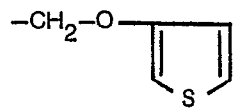 | 0.15 |
| c | 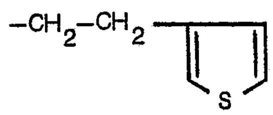 | 0.10 |
| d | 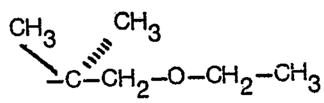 | 0.3 |
| e | $-C-CH_2-O-CH_3$ | 0.27 |
| f | 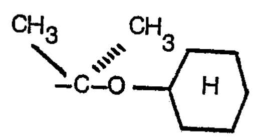 | 0.31 |

Beispiel 18 a

1[4(1Formyl-3-oxo)-butyl]-2[3-tetrahydro-pyranyloxy-1-octenyl]-3-[tetrahydro-pyranyloxy)-5-oxo-cyclopentan (XV)

329 mg 1[6[1,2 - Dihydroxy - 5 - oxo - ) - hexyl] - 2[3 - tetrahydro - pyranyloxy - 1 - octenyl] - 3 - [tetrahydro - pyranyloxy] - 5 - oxo - cyclopentan (XIV) werden in 20 ml Dioxan und 65 ml Wasser gelöst mit 775 mg Natriumperjodat versetzt und 30 Minuten bei Raumtemperatur gerührt. Zur Aufarbeitung wird mit 30 ml ges. NaCl-Lsg. versetzt und mit 4 x 50 ml Äther extrahiert, mit ges. NaCl-Lsg. gewaschen, $MgSO_4$ getrocknet, i.V. eingeengt.
Ausbeute 300 mg
Dieser Aldehyd wurde ohne weitere Reinigung für die nächsten Stufen verwendet.
$R_f$ Wert = 0.48 (Essigester)
NMR: 9.75 (s, 1H) —CHO
5.5 (m, 2H)
4.6 (m, 2H)
4,4—3 (m, 8H)
2,9—2,1 (m, 10H)
2—0.8 (m und 1t, 21H)

Beispiel 18 bis 18 f

In Analogie zu Beispiel 18 a lassen sich aus den Verbindungen 17 b bis 17 f durch selektive Spaltung der Diole die folgenden Verbindungen synthetisieren: (Formel XV):

| Beispiel 18 | $R^2$ | $R_f$-Werte (in Essigester) |
|---|---|---|
| b | 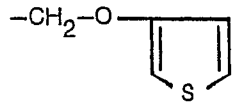 | 0.41 |
| c | 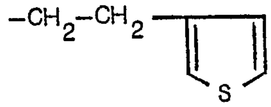 | 0.37 |
| d | 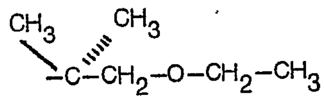 | 0.53 |
| e | 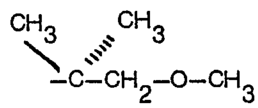 | 0.45 |
| f | 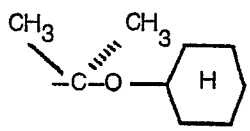 | 0.48 |

Beispiel 19 a

6,9-Dioxo-11,15-bistetrahydro-pyranyloxy-(E)-2, (E)-13-prostadiensäuremethylester (XVI)

300 mg 1[4(1Formyl - 3 - oxo - ) - butyl] - 2[3 - tetrahydropyranyloxy - 1 - octenyl] - 3 - [tetrahydropyranyloxy) - 5 - oxo - cyclopentan (XV) sowie 570 mg Carbomethoxymethylen-triphenylphosphoran (Lit.v. Fasciculus Helv. Chim. acta XL, S. 1247) (1957) werden in 30 ml abs. Toluol unter $N_2$ gerührt. Es wird eingeengt, der Rückstand über eine Säule aus Kieselgel gereinigt. Elutionsmittel Essigester.

Ausbeute: 232 mg gelbes Öl

$R_f$ Wert 0.52 (Essigester)

NMR: 7,2—6,6 (M, 2H)
5,5—6,2 (m, 2H)
4,9—4,5 (m, 2H)
4,4—3,5 (m, 4H)
3,8 (s, 3H)
2,5 (m, 7H)
2—0,8 (m, 28)

Beispiel 19b bis 19f

In Analogie zu Beispiel 19 a lassen sich aus den Verbindungen 18b bis 18f durch Wittig-Reaktion die folgenden Verbindungen darstellen: (Formel XVI)

| Beispiel 19 | $R^2$ | $R_f$-Werte (in Essigester) |
|---|---|---|
| b | 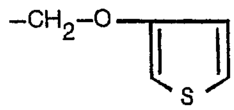 | 0.48 |
| c | 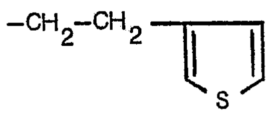 | 0.44 |
| d | 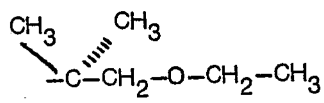 | 0.59 |
| e | 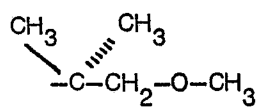 | 0.51 |
| f | 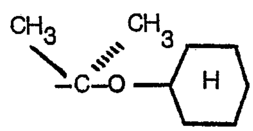 | 0.54 |

### Beispiel 20a

1[4(1-Formyl-3-oxo)-butyl]-2(3-hydroxy-1-octenyl)-3-hydroxy-cyclopentan-5-on (IX)

280 mg 1[4(1 - Formyl - 3 - oxo) - butyl] - 2[3 - tetrahydro - pyranyloxy - 1 - octenyl] - 3 - [tetrahydro - pyranyloxy) - 5 - oxo - cyclopentan (XV) (Beispiel 18a),

5 ml Dimethylglykol, 5 ml Wasser und 1 ml gesättigte Oxalsäurelösung werden 13 Stunden bei 50°C gerührt. Es wird abgekühlt, mit 10 ml gesättigter Natriumchlorid-Lösung versetzt, mit NaCl gesättigt, mit Essigester extrahiert. Die org. Phase wird 1 x mit ges. NaCl-Lösung/Bicarbonatlösung gewaschen, über $MgSO_4$ getrocknet, i.Vak. eingeengt. Über eine Säule aus Kieselgel mit anfangs Äther/Essigester 1:1 als Elutionsmittel, später mit Essigester/Eisessig 99:1 wird von Verunreinigungen abgetrennt.

Ausbeute: 170 mg farbloses Öl

Dünnschichtchromatogramm (Essigsäureäthylester — Eisessig = 97,5:2,5) auf Kieselgelplatten-Merck

$R_f = 0,55$

### Beispiel 20b bis 20f

In Analogie zu Beispiel 20 a lassen sich aus den Verbindungen der beispiele 18b bis 18f durch Abspaltung der Schutzgruppen die folgenden Verbindungen herstellen. (Formel IX):

| Beispiel 20 | $R^2$ | $R_f$-Werte |
|---|---|---|
| b | 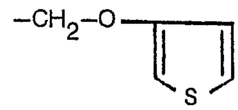 | 0.51 |
| c | 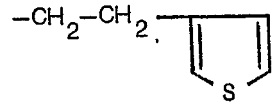 | 0.47 |
| d | 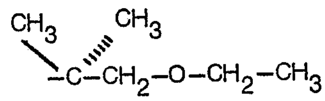 | 0.60 |
| e | 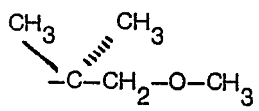 | 0.57 |
| f | 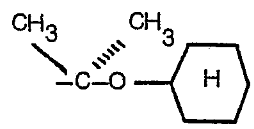 | 0.55 |

### Beispiel 21a

6,9-Dioxo-11,15-dihydroxy-(E)-2,(E)-13-prostadiensäuremethylester (X)

168 mg 6,9 - Dioxo - 11,15 - bistetrahydro - pyranyloxy - (E) - 2, (E) - 13 - prostadiensäuremethylester (XVI), 5 ml Dimethylglykol, 5 ml Wasser und 1 ml gesättigte Oxalsäurelösung werden 13 Stunden bei 50°C gerührt. Es wird abgekühlt, mit 10 ml gesättigter Natriumchlorid-Lösung versetzt, mit NaCl gesättigt, mit Essigester extrahiert. Die org. Phase wird 1 × mit ges. NaCl-Lösung/Bicarbonat-Lösung gewaschen, über $MgSO_4$ getrocknet, i.Vak. eingeengt. Über eine Säule aus Kieselgel mit anfangs Äther/Essigester 1:1 als Elutionsmittel, später mit Essigester wird von Verunreinigungen abgetrennt.

Ausbeute: 109 mg hellgelbes Öl *2 Isomere*
$R_f$-Wert: 0,5 (Essigester:Eisessig= 97,5:2,5)
0,45
NMR: 0.8—2.5 (m, 21) —$CH_2$, —CH
3.0—3.5 (m, 2) —OH,
3.5—4.4 (m, 2) —CH—OH
3.8 (s, 3) $OCH_3$
5.5—6.2 (m, 2) olefin.Prot.
6.6—7.2 (m, 2) olefin.Prot.

### Beispiel 21b bis 21f

In Analogie zu Beispiel 21 a lassen sich die Schutzgruppen aus den Verbindungen der Beispiele 19b bis 19f abspalten, wobei die entsprechenden Verbindungen entstehen.

### Beispiel 22a

6,9,11,15-Tetrahydroxy-(E)-2,(E)-13-prostadiensäuremethylester (VII)

100 mg 6,9-Dioxo-11,15-dihydroxy-(E)-2,(E)-13-prostadiensäuremethylester X werden in 30 ml Methanol und 2 ml Wasser gelöst und bei 0—5°C mit 2 g $NaBH_4$ versetzt. Man rührt bei 0—5°C für 2 weitere Stunden und zersetzt dann das überschüssige $NaBH_4$ mit Essigsäure und fügt 20 ml ges. NaCl-Lsg. hinzu und extrahiert 4 × mit je 30 ml Chloroform, trocknet mit $MgSO_4$ und engt i.Vak. ein. Der Rückstand wird über eine Kieselgelsäule filtriert mit Essigester als Elutionsmittel.

Ausbeute: 90 ml hellgelbes Öl 2 Isomere
$R_f$-Werte 0.26 (Essigester:Eisessig = 9:1)
0.22

NMR:
7,1—6,6 (m, 2H) olefin Protonen
6,1—5,4 (m, 2H) „ „
3,7 (s, 3H) $OCH_3$
4,4—3,5 (m, 4H) —CH—OH
2,6—3,2 (m, 4H) OH
2,5—0,8 (m, 21H) $—CH_2—$, —CH—

Beispiel 22b bis 22f

In Analogie zu Beispiel 22 a lassen sich durch Reduktion aus den Verbindungen der Beispiele 9b bis 9f die folgenden Verbindungen darstellen: (Formel I, $x^1$, $y^1$ und $x^2$, $y^2$ = H bzw. OH):

| Beispiel 22 | $R^2$ |
|---|---|
| b | 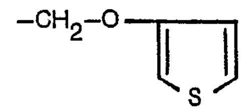 |
| c | 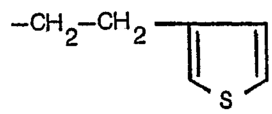 |
| d | 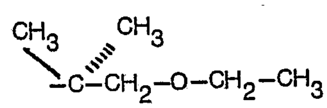 |
| e | 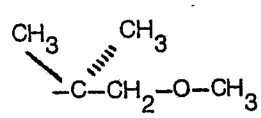 |
| f | 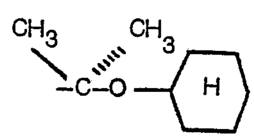 |

**Patentanspruche für die Vertragsstaaten: BE CH DE FR GB IT NL SE**

1. Verbindungen der Formel I

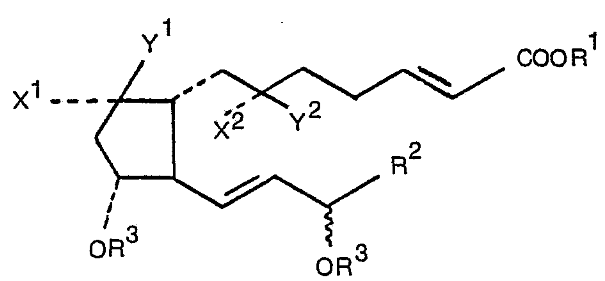

I

worin

$X^1$, $Y^1$ voneinander verschieden sind und H oder OH darstellen,

$X^2$, $Y^2$ voneinander verschieden sind und H oder OH darstellen, oder

$X^1$ und $Y^1$ zusammen sowie $X^2$ und $Y^2$ zusammen jeweils Sauerstoff bedeuten,

$R^1$ a) Wasserstoff oder einen geradkettigen, verzweigten, gesättigten oder ungesättigten aliphatischen oder cycloaliphatischen Kohlenwasserstoff-Rest mit bis zu zehn Kohlenstoffatomen oder

b) ein physiologisch verträgliches Metall- oder $NH_4^+$-Ion oder ein Ammoniumion, das sich von einem primären, sekundären der tertiären Amin ableitet, bedeutet,

$R^2$ einen geraden oder verzweigten Alkylrest mit eines bis sieben Kohlenstoffatomen bedeutet, in dem eine nicht endständige $CH_2$-Gruppe durch ein Sauerstoffatom ersetzt ist, oder der substituiert ist

a) mit einem $\alpha$- oder $\beta$-Theinyl- oder -Furylrest, die ihrerseits im Kern 1—3 fach substituiert sein können mit Halogen, Trifluormethyl, und/oder Alkyl oder Alkoxy mit je 1—6 C-Atomen, oder

b) mit einem α- oder β-Thienyloxyrest oder einem Cycloalkoxyrest mit 3 bis 7 Kohlenstoffatomen, wobei die genannten Reste ihrerseits im Kern 1—3 fach substituiert sein können mit Halogen, Trifluormethyl und/oder Alkyl oder Alkoxy mit je 1—6 C-Atomen, und

$R^3$ Wasserstoff oder eine leicht abspaltbare Schutzgruppe bedeutet.

2. Verbindungen der Formel I gemäß Anspruch 1, dadurch gekennzeichnet, daß $X^1$ und $Y^1$ zusammen sowie $X^2$ und $Y^2$ zusammen jeweils Sauerstoff darstellen und $R^3$ Wasserstoff bedeutet.

3. Verfahren zur herstellung von Verbindungen der Formel I

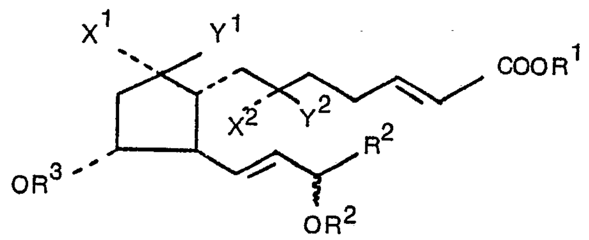

I

worin $R^1$, $R^2$, $R^3$, $X^1$, $Y^1$, $X^2$ und $Y^2$ die zur Formel I in Anspruch 1 genannten Bedeutungen haben dadurch gekennzeichnet, daß man eine Verbindung der formel XV

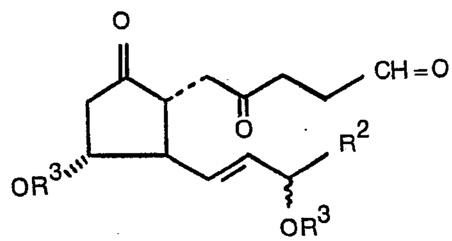

XV

worin $R^2$ und $R^3$ die zur Formel I genannte Bedeutung haben, mit dem Ylid der Formel VI

$$(R^4)_3P{=}CH{-}COOCH_3 \qquad VI$$

worin die Reste $R^4$ gleich oder verschieden sind und geradkettiges ($C_1$—$C_4$)-Alkyl oder Phenyl bedeuten, zu einem Ester der Formel I, worin $R^1$ die Methylgruppe bedeutet, umsetzt, gegebenenfalls in dem erhaltenen Ester der Formel I, worin $R^1$ die Methylgruppe und $R^3$ leicht abspaltbare Schutzgruppen bedeuten, durch saure Hydrolyse die Schutzgruppen abspaltet, gegebenenfalls den Ester der Formel I, worin $R^1$ die Methylgruppe darstellt, durch Hydrolyse überführt in eine Verbindung der Formel I, worin $R^1$ Wasserstoff bedeutet, gegebenenfalls eine Verbindung der Formel I, worin $R^1$, $R^2$ und $R^3$ die zur Formel I in Anspruch 1 genannten Bedeutung haben und $X^1$ und $Y^1$ zusammen sowie $X^2$ und $Y^2$ zusammen jeweils Sauerstoff bedeuten, reduziert zu einer Verbindung der Formel I, worin $X^1$ und $Y^1$ jeweils verschieden sind und H oder OH bedeuten, sowie $X^2$ und $Y^2$ jeweils verschieden sind und H oder OH bedeuten, gegebenenfalls eine Verbindung der Formel I, worin $X^1$, $X^2$, $Y^1$, $Y^2$, $R^2$ und $R^3$ die zur Formel I in Anspruch 1 angegebene Bedeutung haben und $R^1$ Wasserstoff bedeutet, zu einer Verbindung der Formel I, worin $X^1$, $X^2$, $Y^1$, $Y^2$, $R^2$ und $R^3$ die zur Formel I in Anspruch 1 angegebenen Bedeutungen haben, wobei $R^1$ jedoch nicht Wasserstoff bedeutet, verestert, und gegebenenfalls eine Verbindung der Formel I, worin $X^1$, $X^2$, $Y^1$, $Y^2$ $R^2$ und $R^3$ die zur Formel I in Anspruch 1 angegebenen Bedeutungen haben und $R^1$ Wasserstoff bedeutet, in eine physiologisch verträgliches Metall- oder Aminsalz überführt.

4. Verfahren zur Herstellung von Verbindungen der Formel I

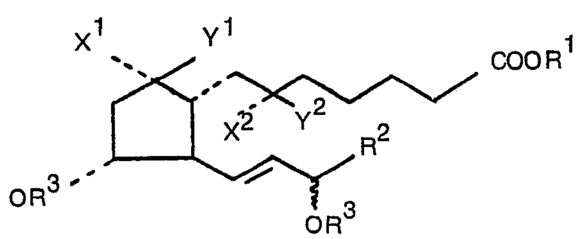

I

worin $X^1$, $Y^1$, $X^2$, $Y^2$, $R^1$, $R^2$ und $R^3$ die zur Formel I in Anspruch 1 angegebenen Bedeutungen haben, dadurch gekennzeichnet, daß man

a) ein Lactol der Formel II

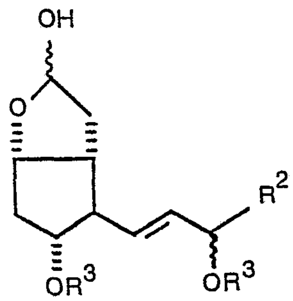

II

worin $R^2$ und $R^3$ die zur Formel I angegebene Bedeutung haben, mit einer Grignard-Verbindung der Formel XI

$$HalMg—CH_2—CH_2—CH=CH_2 \quad XI$$

worin Hal, Chlor, Brom oder Jod bedeutet, umsetzt zu einem Alkohol der Formel XII

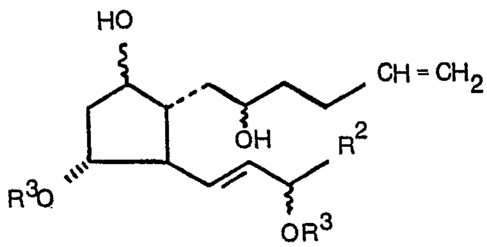

XII

worin $R^2$ und $R^3$ die zur Formel I angegebene Bedeutung haben,

b) den erhaltenen Alkohol der Formel XII oxidiert, wobei ein Keton der Formel XIII

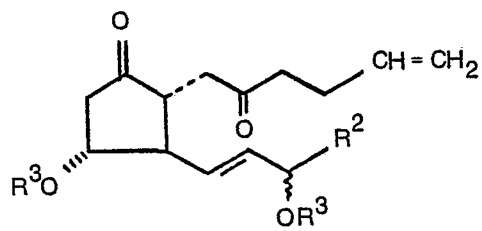

XIII

worin $R^2$ und $R^3$ die in Formel I angegebene Bedeutung haben, entsteht,

c) in dem erhaltenen Diketon der Formel XIII die endständige Doppelbindung selektiv oxidiert, wobei ein Diol der Formel XIV

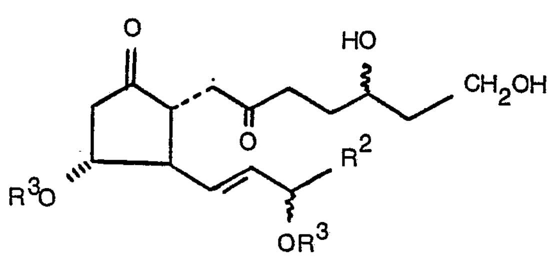

XIV

worin $R^2$ und $R^3$ die zur Formel I angegebene Bedeutung haben, entsteht,

d) das erhaltene Diol der Formel XIV oxidiert zu einem Diketoaldehyd der Formel XV

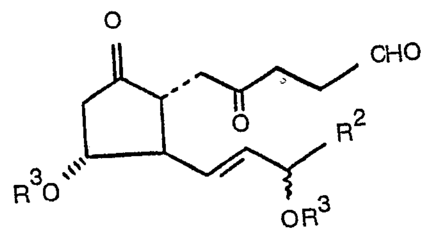

XV

worin $R^2$ und $R^3$ die in Formel I angegebene Bedeutung haben,

e) den erhaltenen Diketoaldehyd der Formel XV umsetzt mit dem Ylid der Formel VI

$$(R^4)_3P{=}CH{-}COOCH_3 \qquad VI$$

wobei die Reste $R^4$ gleich oder verschieden sind und geradkettiges $(C_1{-}C_4)$-Alkyl oder Phenyl bedeuten, zu einer Verbindung der Formel XVI

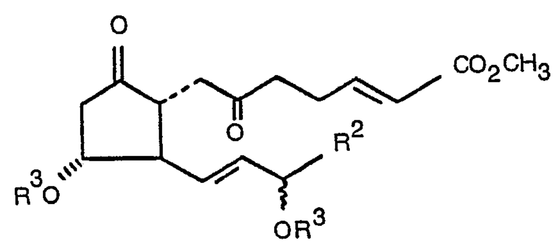

XVI

worin $R^2$ und $R^3$ die in Formel I angegebene Bedeutung haben, oder

$f_1$) in der Verbindung der Formel XV durch saure Hydrolyse die Schutzgruppen abspaltet, wobei ein Aldehyd der Formel IX

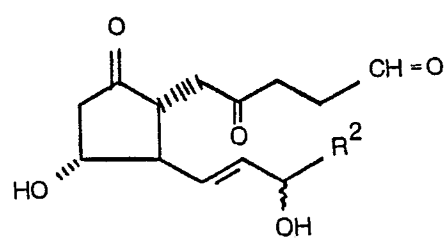

IX

worin $R^2$ die in Formel I angegebene Bedeutung hat, entsteht, und

$f_2$) den Aldehyd der Formel IX umsetzt mit dem Ylid der Formel VI

$$(R^4)_3P{=}CH{-}COOCH_3 \qquad VI$$

worin die Reste $R^4$ gleich oder verschieden sein können und geradkettiges $(C_1{-}C_4)$-Alkyl oder Phenyl bedeuten, zu einer Verbindung der Formel X

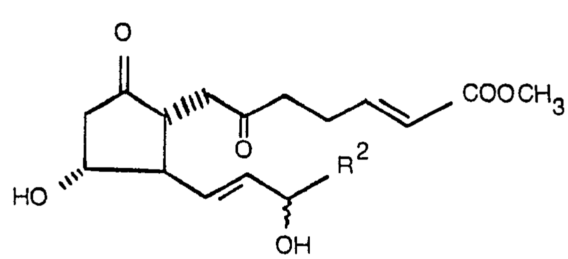

X

worin $R^2$ die in Formel I angegebene Bedeutung hat, oder

g) gewünschtenfalls in einer Verbindung der Formel

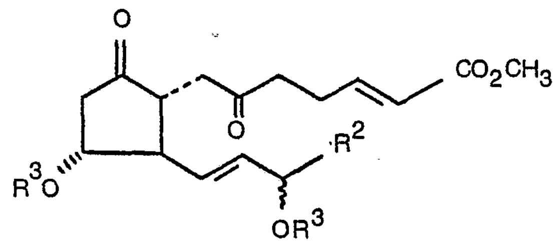

XVI

worin $R^2$ und $R^3$ die zur Formel I angegebene Bedeutung haben, durch saure Hydrolyse die Schutzgruppen $R^3$ abspaltet, wobei eine Verbindung der Formel X entsteht, und

h) gewünschtenfalls eine Verbindung der Formel X mit einem komplexen Metallhydrid reduziert zu einer Verbindung der Formel VII

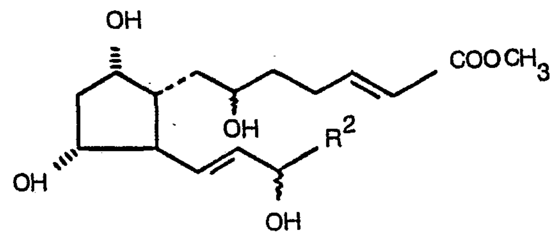

VII

i) gewünschtenfalls eine Verbindung der Formel VII, X oder XVI durch Hydrolyse überführt in eine Verbindung der Formel I, worin $R^1$ Wasserstoff bedeutet und $X^1$, $Y^1$, $X^2$, $Y^2$, $R^2$ und $R^3$ die zu Formel I angegebene Bedeutung haben,

k) gewünschtenfalls eine Verbindung der Formel I, worin $R^1$ und $R^3$ Wasserstoff bedeuten, $R^2$ die zu Formel I genannte Bedeutung hat und $X^1$ und $Y^1$ sowie $X^2$ und $Y^2$ jeweils zusammen Sauerstoff bedeuten, mit einem komplexen Metallhydrid reduziert zu einer Verbindung der Formel I, worin $X^1$ und $Y^1$ sowie $X^2$ und $Y^2$ jeweils voneinander verschieden sind und Wasserstoff oder Hydroxyl bedeuten,

l) gewünschtenfalls eine Verbindung der Formel I, worin $X^1$, $X^2$, $Y^1$, $Y^2$, $R^2$ und $R^3$ die zu Formel I angegebene Bedeutung haben, und $R^1$ Wasserstoff bedeutet, zu einer Verbindung der Formel I, worin $X^1$, $X^2$, $Y^1$, $Y^2$, $R^2$ und $R^3$ die zu Formel I angegebenen Bedeutungen haben, wobei $R^1$ jedoch nicht Wasserstoff bedeutet, verestert, und

m) gewünschtenfalls eine Verbindung der Formel I, worin $X^1$, $X^2$, $Y^1$, $Y^2$, $R^2$ und $R^3$ die zu Formel I angegebenen Bedeutungen haben, und $R^1$ Wasserstoff bedeutet, in ein physiologisch verträgliches Metall- oder Aminsalz überführt.

5. Arzneimittel, gekennzeichnet durch einen Gehalt an einer Verbindung der Formel I gemäß Anspruch 1.

**Patentansprüche für den Vertragsstaat: AT**

1. Verfahren zur Herstellung von Verbindungen der Formel I

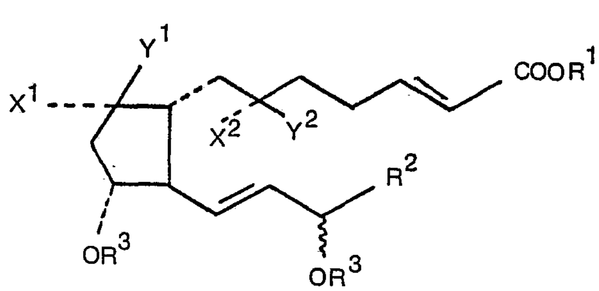

I

worin

$X^1$, $Y^1$ voneinander verschieden sind und H oder OH darstellen,

$X^2$, $Y^2$ voneinander verschieden sind und H oder OH darstellen, oder $X^1$ und $Y^1$ zusammen sowie $X^2$ und $Y^2$ zusammen jeweils Sauerstoff bedeuten,

$R^1$ a) Wasserstoff oder einen geradkettigen, verzweigten, gesättigten oder ungesättigten aliphatischen oder cycloaliphatischen Kohlenwasserstoff-Rest mit bis zu zehn Kohlenstoffatomen oder

b) ein physiologisch verträglisches Metall- oder $NH_4^+$-Ion oder ein Ammoniumion, das sich von einem primären, sekundären der tertiären Amin ableitet, bedeutet,

$R^2$ einen geraden oder verzweigten Alkylrest mit eines bis sieben Kohlenstoffatomen bedeutet, in dem eine nicht endständige $CH_2$-Gruppe durch ein Sauerstoffatom ersetzt ist, oder der substituiert ist

a) mit einem $\alpha$- oder $\beta$-Theinyl- oder -Furylrest, die ihrerseits im Kern 1—3 fach substituiert sein können mit Halogen, Trifluormethyl, und/oder Alkyl oder Alkoxy mit je 1—6 C-Atomen, oder

b) mit einem $\alpha$- oder $\beta$-Thienyloxyrest oder einem Cycloalkoxyrest mit 3 bis 7 Kohlenstoffatomen, wobei die genannten Reste ihrerseits im Kern 1—3 fach substituiert sein können mit Halogen, Trifluormethyl und/oder Alkyl oder Alkoxy mit je 1—6 C-Atomen, und

$R^3$ Wasserstoff oder eine leicht abspaltbare Schutzgruppe bedeutet, dadurch gekennzeichnet, daß eine Verbindung der Formel XV

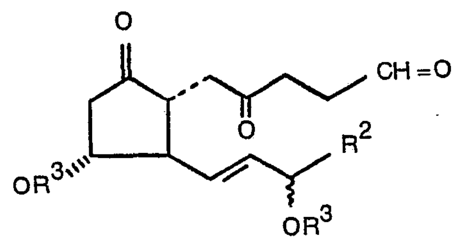

XV

worin $R^2$ und $R^3$ die zur Formel I genannte Bedeutung haben, mit dem Ylid der Formel VI

$$(R^4)_3P{=}CH{—}COOCH_3 \qquad VI$$

worin die Reste $R^4$ gleich oder verschieden sind und geradkettiges ($C_1$—$C_4$)-Alkyl oder Phenyl bedeuten, zu einem Ester der Formel I, worin $R^1$ die Methylgruppe bedeutet, umsetzt, gegebenenfalls in dem erhaltenen Ester der Formel I, worin $R^1$ die Methylgruppe und $R^3$ leicht abspaltbare Schutzgruppen bedeuten, durch saure Hydrolyse die Schutzgruppen abspaltet, gegebenenfalls den Ester der Formel I, worin $R^1$ die Methylgruppe darstellt, durch Hydrolyse überführt in eine Verbindung der Formel I, worin $R^1$ Wasserstoff bedeutet, gegebenenfalls eine Verbindung der Formel I, worin $R^1$, $R^2$ und $R^3$ die oben zur Formel I genannten Bedeutungen haben und $X^1$ und $Y^1$ zusammen sowie $X^2$ und $Y^2$ zusammen jeweils Sauerstoff bedeuten, reduziert zu einer Verbindung der Formel I, worin $X^1$ und $Y^1$ jeweils verschieden sind und H oder OH bedeuten, sowie $X^2$ und $Y^2$ jeweils verschieden sind und H oder OH bedeuten, gegebenenfalls eine Verbindung der Formel I, worin $X^1$, $X^2$, $Y^1$, $Y^2$, $R^2$ und $R^3$ die oben zur Formel I angegebene Bedeutung haben und $R^1$ Wasserstoff bedeutet, zu einer Verbindung der Formel I, worin $X^1$, $X^2$, $Y^1$, $Y^2$, $R^2$ und $R^3$ die oben zur Formel I angegebenen Bedeutungen haben, wobei $R^1$ jedoch nicht Wasserstoff bedeutet, verestert, und gegebenenfalls eine Verbindung der Formel I, worin $X^1$, $X^2$, $Y^1$, $Y^2$ $R^2$ und $R^3$ die oben zur Formel I in angegebenen Bedeutungen haben und $R^1$ Wasserstoff bedeutet, in eine physiologisch verträgliches Metall- oder Aminsalz überführt.

2. Verfahren zur herstellung von Verbindungen der Formel I

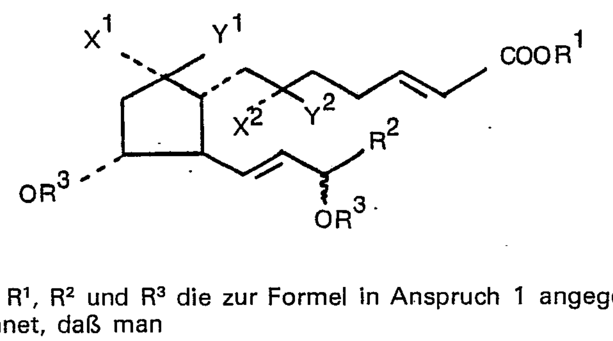

I

worin $X^1$, $Y^1$, $X^2$, $Y^2$, $R^1$, $R^2$ und $R^3$ die zur Formel in Anspruch 1 angegebenen Bedeutungen haben, dadurch gekennzeichnet, daß man

a) ein Lactol der Formel II

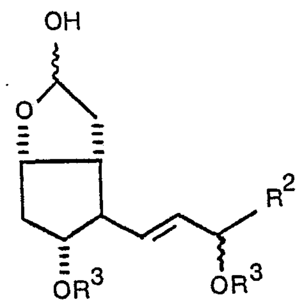

II

worin $R^2$ und $R^3$ die zur Formel I angegebene Bedeutung haben, mit einer Grignard-Verbindung der Formel XI

$$HalMg—CH_2—CH_2—CH{=}CH_2 \qquad XI$$

worin Hal Chlor, Brom oder Jod bedeutet, umsetzt zu einem Alkohol der Formel XII

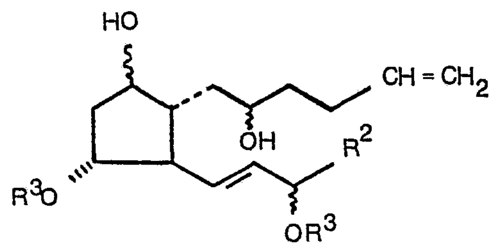

XII

worin $R^2$ und $R^3$ die zur Formel I angegebene Bedeutung haben,

b) den erhaltenen Alkohol der Formel XII oxidiert, wobei ein Keton der Formel XIII

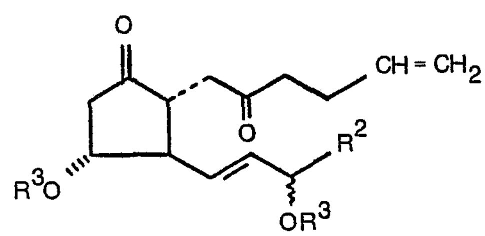

XIII

worin $R^2$ und $R^3$ die in Formel I angegebene Bedeutung haben, entsteht,

c) in dem erhaltenen Diketon der Formel XIII die endständige Doppelbindung selektiv oxidiert, wobei ein Diol der Formel XIV

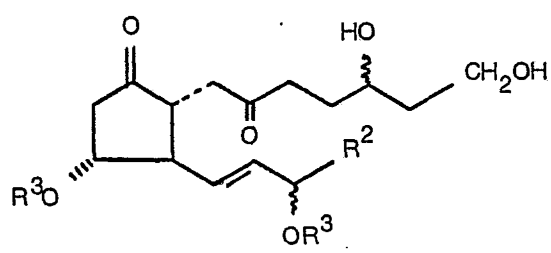

XIV

worin $R^2$ und $R^3$ die zur Formel I angegebene Bedeutung haben, entsteht,

d) das erhaltene Diol der Formel XIV oxidiert zu einem Diketoaldehyd der Formel XV

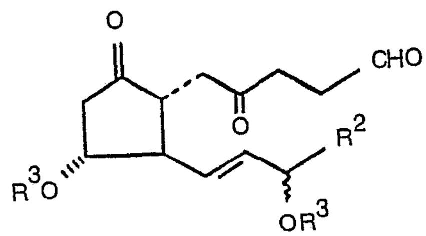

XV

worin $R^2$ und $R^3$ die in Formel I angegebene Bedeutung haben,

e) den erhaltenen Diketoaldehyd der Formel XV umsetzt mit dem Ylid der Formel VI

$$(R^4)_3P{=}CH{-}COOCH_3 \qquad VI$$

wobei die Reste $R^4$ gleich oder verschieden sind und geradkettiges ($C_1$—$C_4$)-Alkyl oder Phenyl bedeuten, zu einer Verbindung der Formel XVI

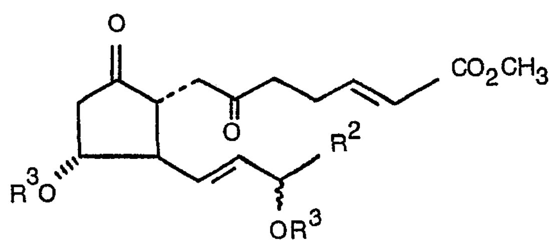

XVI

worin $R^2$ und $R^3$ die in Formel I angegebene Bedeutung haben, oder

$f_1$) in der Verbindung der Formel XV durch saure Hydrolyse die Schutzgruppen abspaltet, wobei ein Aldehyd der Formel IX

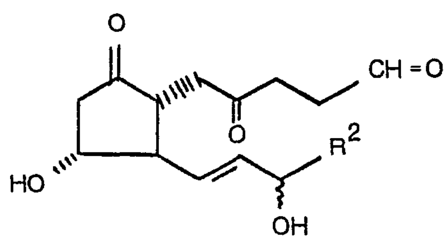

IX

worin $R^2$ die in Formel I angegebene Bedeutung hat, entsteht, und

$f_2$) den Aldehyd der Formel IX umsetzt mit dem Ylid der Formel VI

$$(R^4)_3P{=}CH{-}COOCH_3 \qquad VI$$

worin die Reste $R^4$ gleich oder verschieden sein können und geradkettiges ($C_1$—$C_4$)-Alkyl oder Phenyl bedeuten, zu einer Verbindung der Formel X

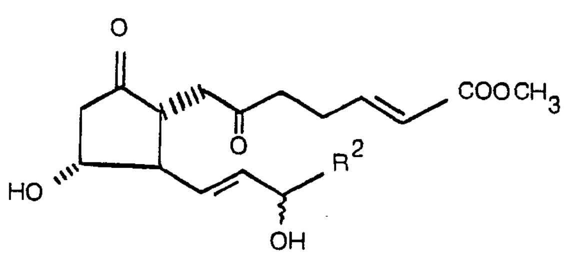

X

worin $R^2$ die in Formel I angegebene Bedeutung hat, oder

g) gewünschtenfalls in einer Verbindung der Formel

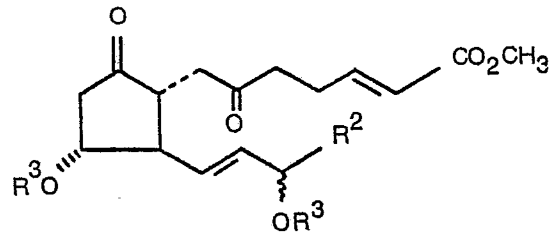

XVI

worin $R^2$ und $R^3$ die zur Formel I angegebene Bedeutung haben, durch saure Hydrolyse die Schutzgruppen $R^3$ abspaltet, wobei eine Verbindung der Formel X entsteht, und

h) gewünschtenfalls eine Verbindung der Formel X mit einem komplexen Metallhydrid reduziert zu einer Verbindung der Formel VII

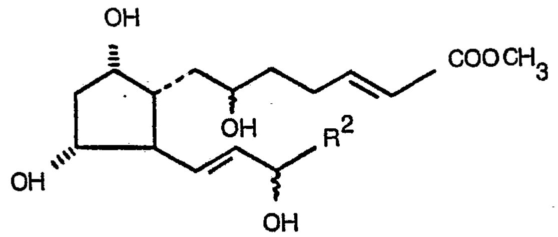

VII

i) gewünschtenfalls eine Verbindung der Formel VII, X oder XVI durch Hydrolyse überführt in eine Verbindung der Formel I, worin $R^1$ Wasserstoff bedeutet und $X^1$, $Y^1$, $X^2$, $Y^2$, $R^2$ und $R^3$ die zu Formel I angegebene Bedeutung haben,

k) gewünschtenfalls eine Verbindung der Formel I, worin $R^1$ und $R^3$ Wasserstoff bedeuten, $R^2$ die zu Formel I genannte Bedeutung hat und $X^1$ und $Y^1$ sowie $X^2$ und $Y^2$ jeweils zusammen Sauerstoff bedeuten, mit einem komplexen Metallhydrid reduziert zu einer Verbindung der Formel I, worin $X^1$ und $Y^1$ sowie $X^2$ und $Y^2$ jeweils voneinander verschieden sind und Wasserstoff oder Hydroxyl bedeuten,

l) gewünschtenfalls eine Verbindung der Formel I, worin $X^1$, $X^2$, $Y^1$, $Y^2$, $R^2$ und $R^3$ die zu Formel I angegebene Bedeutung haben, und $R^1$ Wasserstoff bedeutet, zu einer Verbindung der Formel I, worin $X^1$, $X^2$, $Y^1$, $Y^2$, $R^2$ und $R^3$ die zu Formel I angegebenen Bedeutungen haben, wobei $R^1$ jedoch nicht Wasserstoff bedeutet, verestert, und

m) gewünschtenfalls eine Verbindung der Formel I, worin $X^1$, $X^2$, $Y^1$, $Y^2$, $R^2$ und $R^3$ die zu Formel I angegebenen Bedeutungen haben, und $R^1$ Wasserstoff bedeutet, in ein physiologisch verträgliches Metall- oder Aminsalz überführt.

3. Verfahren zur Herstellung von Arzneimitteln, dadurch gekennzeichnet, daß man eine Verbindung der Formel I gemäß Anspruch 1, gebenenfalls mit pharmazeutisch üblichen Trägerstoffen und-oder Stabilisatoren in eine therapeutisch geeignete Anwendungsform bringt.

**Claims for the Contracting States: BE CH DE FR GB IT NL SE**

1. Compounds of the formula I

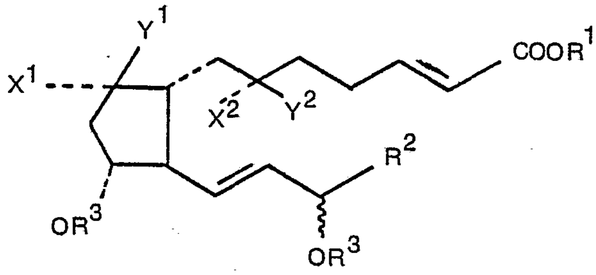

I

in which

$X^1$ and $Y^1$ as well as $X^2$ and $Y^2$ together denote oxygen or are different and individually are hydrogen or hydroxyl;

$R^1$ denotes a) hydrogen or a linear or branched, saturated or unsaturated, aliphatic or cycloaliphatic hydrocarbon radial having up to 10 carbon atoms; or

b) a physiologically acceptable metal ion or $NH_4^+$-ion, or an ammonium ion derived from a primary, secondary or tertiary amine;

$R^2$ denotes a linear or branched alkyl radical having from 1 to 7 carbon atoms in which a non-terminal $CH_2$ group can be replaced by oxygen, or which can be substituted with

a) halogen or an $\alpha$- or $\beta$-thienyl or furyl radical which, on their part, can be substituted 1 to 3 times in the nucleus by halogen, trifluoromethyl, and/or alkyl or alkoxy each having from 1 to 6 carbon atoms, or

b) with an $\alpha$- or $\beta$-thienyloxy or a cycloalkoxy radical having from 3 to 7 carbon atoms, which radicals may be substituted 1 to 3 times in the nucleus with halogen, trifluoromethyl and/or alkyl or alkoxy each having from 1 to 6 carbon atoms,

$R^3$ denotes hydrogen or an easily detachable protective group.

2. Compounds of the formula I as claimed in Claim 1, wherein $X^1$ and $Y^1$ as well as $X^2$ and $Y^2$ together denote oxygen and $R^3$ denotes hydrogen.

3. Process for the manufacture of compounds of the formula I

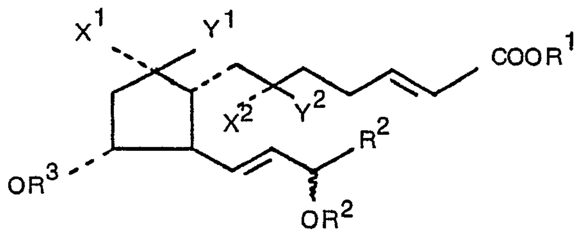

I

in which $R^1$, $R^2$, $R^3$, $X^1$, $Y^1$, $X^2$ and $Y^2$ are as defined under formula I in Claim 1, which comprises reacting a compound of the formula XV

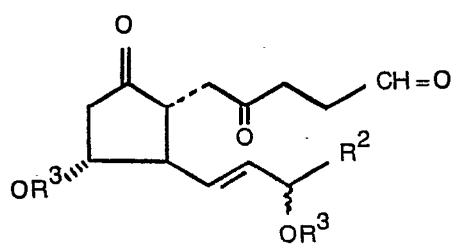

XV

in which $R^2$ and $R^3$ are as defined under formula I with an ylide of the formula VI

$$(R^4)_3P=CH-COOCH_3 \qquad VI$$

in which the radicals $R^4$, which are identical or different, denote linear $C_1$—$C_4$ alkyl or phenyl to give an ester of the formula I in which $R^1$ denotes methyl; in the ester of the formula I obtained, in which $R^1$ denotes methyl and $R^3$ denotes easily detachable protective groups, optionally detaching the said protective groups by acid hydrolysis; optionally converting the ester of the formula I, in which $R^1$ denotes methyl, by hydrolysis into a compound of the formula I in which $R^1$ denotes hydrogen; optionally reducing a compound of the formula I in which $R^1$, $R^2$ and $R^3$ are as defined under formula I in Claim 1 and $X^1$ and $Y^1$ as well as $X^2$ and $Y^2$ each time together denote oxygen, to give a compound of the formula I in which $X^1$ and $Y^1$ as well as $X^2$ and $Y^2$ are different and denote hydrogen or hydroxy; optionally esterifying a compound of the formula I in which $X^1$, $X^2$, $Y^1$, $Y^2$, $R^2$ and $R^3$ are as defined under formula I in Claim 1 and $R^1$ denotes hydrogen to give a compound of the formula I in which $X^1$, $X^2$, $Y^1$, $Y^2$, $R^2$ and $R^3$ are as defined under formula I in Claim 1 and $R^1$ is not hydrogen; and optionally converting a compound of the formula I in which $X^1$, $X^2$, $Y^1$, $Y^2$ and $R^2$ and $R^3$ are as defined under formula I in Claim 1 and $R^1$ denotes hydrogen into a physiologically acceptable metal or amine salt.

4. Process for the manufacture of compounds of the formula I

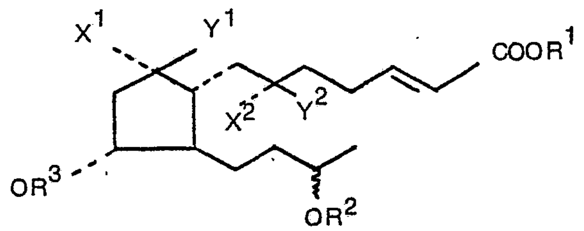

I

in which $X^1$, $Y^1$, $X^2$, $Y^2$, $R^1$, $R^2$ and $R^3$ are as defined under formula I in Claim 1 which comprises
a) reacting a lactol of the formula II

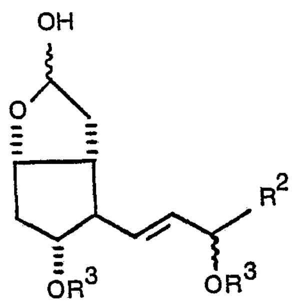

II

in which $R^2$ and $R^3$ are as defined under formula I with a Grignard compound of the formula XI

$$HalMg—CH_2—CH_2—CH=CH_2 \qquad XI$$

in which Hal denotes chlorine, bromine or iodine to give an alcohol of the formula XII

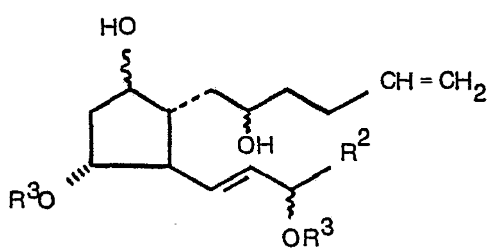

XII

in which $R^2$ and $R^3$ are as defined under formula I,
b) oxidizing the alcohol of the formula XII obtained to give a ketone of the formula XIII

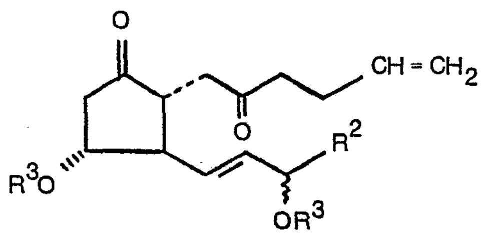

XIII

in which $R^2$ and $R^3$ are as defined in formula I,
c) in the diketone of the formula XIII obtained selectively oxidizing the terminal double bond whereupon a diol of the formula XIV

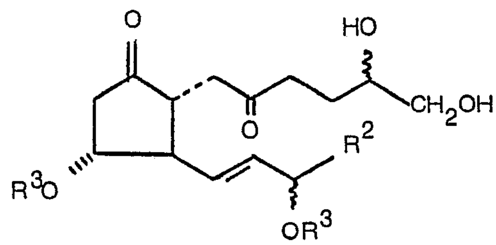

XIV

in which $R^2$ and $R^3$ are as defined under formula I is obtained,

d) oxidizing the diol of the formula XIV obtained to give a diketoaldehyde of the formula XV

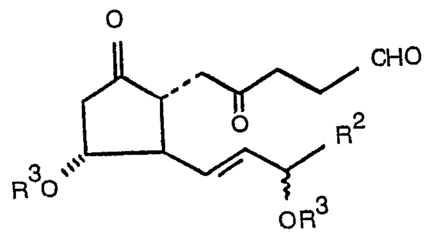

XV

in which $R^2$ and $R^3$ are as defined under formula I,

e) reacting the diketoaldehyde of the formula XV obtained with the ylide of the formula VI

$$(R^4)_3P = CH - COOCH_3 \qquad VI$$

in which the radicals $R^4$, which are identical or different, denote linear $C_1$—$C_4$ alkyl or phenyl to give a compound of the formula XVI

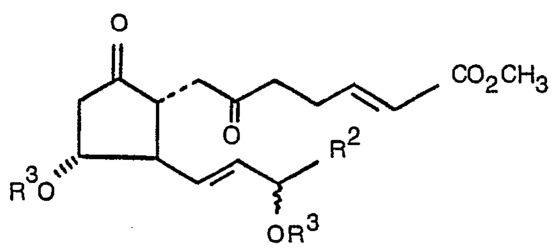

XVI

in which $R^2$ and $R^3$ are as defined under formula I, or

$f_1$) in the compound of the formula XV detaching the protective groups by acid hydrolysis, whereupon an aldehyde of the formula IX is obtained

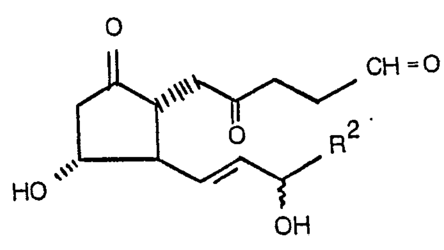

IX

in which $R^2$ is as defined under formula I, and

$f_2$) reacting the aldehyde of the formula IX with the ylide of the formula VI

$$(R^4)_3P = CH - COOCH_3 \qquad VI$$

in which the radicals $R^4$, which are identical or different, denote linear $C_1$—$C_4$ alkyl or phenyl to give a compound of the formula X

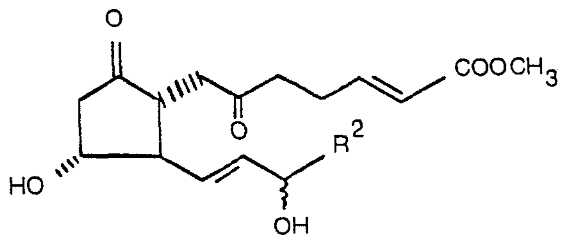

X

in which $R^2$ is as defined under formula I, or

g) in a compound of the formula XVI

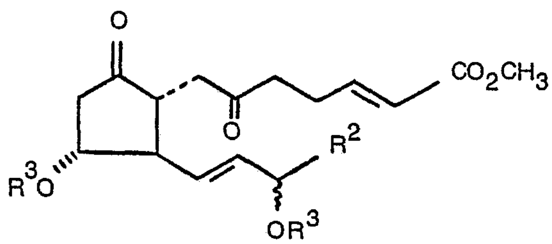

XVI

in which $R^2$ and $R^3$ are as defined under formula I optionally detaching the protective groups $R^3$ by acid hydrolysis, whereupon a compound of the formula X is obtained;

h) optionally reducing a compound of the formula X with a complex metal hydride to give a compound of the formula VII,

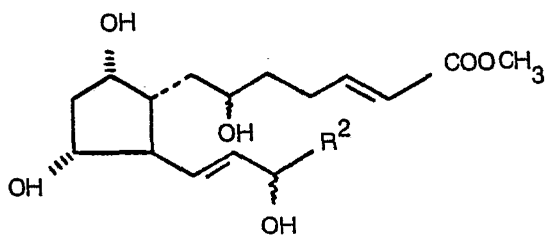

VII

i) optionally converting a compound of the formula VII, X or XVI by hydrolysis into a compound of the formula I in which $R^1$ denotes hydrogen and $X^1$, $Y^1$, $X^2$, $Y^2$, $R^2$ and $R^3$ are as defined under formula I;

k) optionally reducing a compound of the formula I, in which $R^1$ and $R^3$ denote hydrogen, $R^2$ is as defined under formula I and $X^1$ and $Y^1$ as well as $X^2$ and $Y^2$ together denote oxygen with a complex metal hydride to give a compound of the formula I in which $X^1$ and $Y^1$ as well as $X^2$ and $Y^2$ are different from each other and denote hydrogen or hydroxy;

l) optionally esterifying a compound of the formula I in which $X^1$, $X^2$, $Y^1$, $Y^2$, $R^2$ and $R^3$ are as defined under formula I and $R^1$ denotes hydrogen to give a compound of the formula I in which $X^1$, $X^2$, $Y^1$, $Y^2$, $R^2$ and $R^3$ are as defined under formula I and $R^1$ is not hydrogen, and

m) optionally converting a compound of the formula I in which $X^1$, $X^2$, $Y^1$, $Y^2$, $R^2$ and $R^3$ are as defined under formula I and $R^1$ denotes hydrogen into a physiologically acceptable metal or amine salt.

5. A pharmaceutical composition which comprises an effective amount of a compound of the formula I claimed in Claim 1.

**Claims for the Contracting State: AT**

1. A process for the manufacture of compounds of the formula I

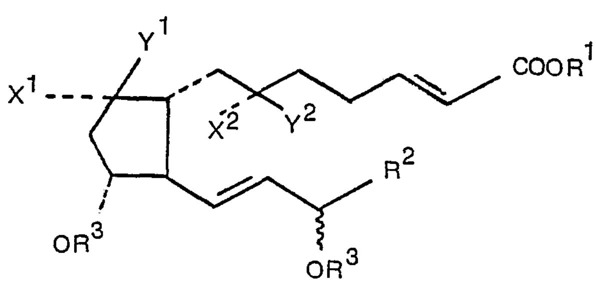

I

in which

$X^1$ and $Y^1$ as well as $X^2$ and $Y^2$ together denote oxygen or are different and individually are hydrogen or hydroxyl;

$R^1$ denotes a) hydrogen or a linear or branched, saturated or unsaturated, aliphatic or cycloaliphatic hydrocarbon radical having up to 10 carbon atoms; or

b) a physiologically acceptable metal ion or $NH_4^+$-ion, or an ammonium ion derived from a primary, secondary or tertiary amine;

$R^2$ denotes a linear or branched alkyl radical having from 1 to 7 carbon atoms in which a non-terminal $CH_2$ group can be replaced by oxygen, or which can be substituted with

a) an $\alpha$- or $\beta$-thienyl or furyl radical which, on their part, can be substituted 1 to 3 times in the nucleus by halogen, trifluoromethyl, and/or alkyl or alkoxy each having from 1 to 6 carbon atoms, or

b) and $\alpha$- and $\beta$-thienyloxy or a cycloalkoxy radical having from 3 to 7 carbon atoms, which radicals may be substituted 1 to 3 times in the nucleus with the halogen, trifluoromethyl and/or alkyl or alkoxy each having from 1 to 6 carbon atoms,

$R^3$ denotes hydrogen or an easily detachable protective group, which comprises reacting a compound of the formula XV

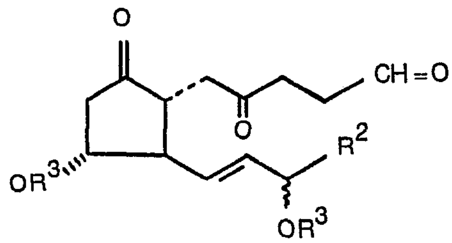

XV

in which $R^2$ and $R^3$ are as defined under formula I with an ylide of the formula VI

$$(R^4)_3P = CH - COOCH_3 \qquad VI$$

in which the radicals $R^4$, which are identical or different, denote linear $C_1$—$C_4$ alkyl or phenyl to give an ester of the formula I in which $R^1$ denotes methyl; in the ester of the formula I obtained, in which $R^1$ denotes methyl and $R^3$ denotes easily detachable protective groups, optionally detaching the said protective groups by acid hydrolysis; optionally converting the ester of the formula I, in which $R^1$ denotes methyl, by hydrolysis into a compound of the formula I in which $R^1$ denotes hydrogen; optionally reducing a compound of the formula I in which $R^1$, $R^2$ and $R^3$ are as defined under formula I and $X^1$ and $Y^1$ as well as $X^2$ and $Y^2$ each time together denote oxygen, to give a compound of the formula I in which $X^1$ and $Y^1$ as well as $X^2$ and $Y^2$ are different and denote hydrogen or hydroxy; optionally esterifying a compound of the formula I in which $X^1$, $X^2$, $Y^1$, $Y^2$, $R^2$ and $R^3$ are as defined under formula I and $R^1$ denotes hydrogen to give a compound of the formula I in which $X^1$, $X^2$, $Y^1$, $Y^2$, $R^2$ and $R^3$ are as defined under formula I and $R^1$ is not hydrogen; and optionally converting a compound of the formula I in which $X^1$, $X^2$, $Y^1$, $Y^2$ and $R^2$ and $R^3$ are as defined under formula I and $R^1$ denotes hydrogen into physiologically acceptable metal or amine salt.

2. Process for the manufacture of compounds of the formula I

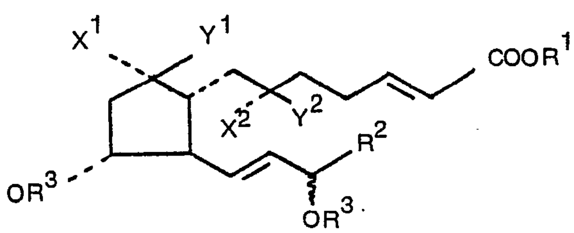

I

in which $X^1$, $Y^1$, $X^2$, $Y^2$, $R^1$, $R^2$ and $R^3$ are as defined under formula I in Claim 1 which comprises

a) reacting a lactol of the formula II

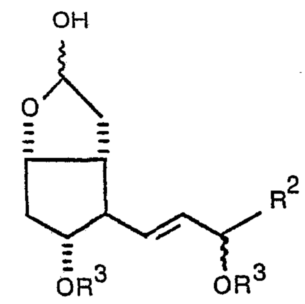

II

in which $R^2$ and $R^3$ are as defined under formula I with a Grignard compound of the formula XI

$$HalMg—CH_2—CH_2—CH=CH_2 \quad XI$$

in which Hal denotes chlorine, bromine or iodine to give an alcohol of the formula XII

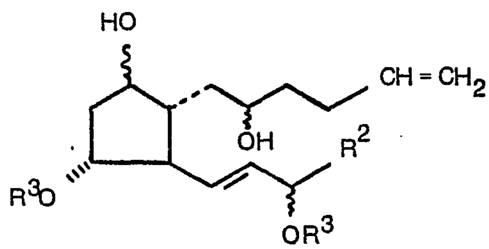

XII

in which $R^2$ and $R^3$ are as defined under formula I,

b) oxidizing the alcohol of the formula XII obtained to give a ketone of the formula XIII

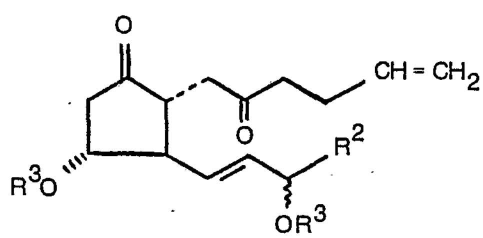

XIII

in which $R^2$ and $R^3$ are as defined in formula I,

c) in the diketone of the formula XIII obtained selectively oxidizing the terminal double bond whereupon a diol of the formula XIV

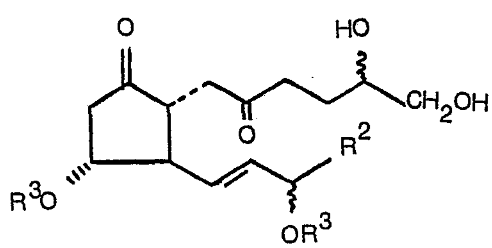

XIV

in which $R^2$ and $R^3$ are as defined under formula I is obtained.

d) oxidizing the diol of the formula XIV obtained to give a diketoaldehyde of the formula XV

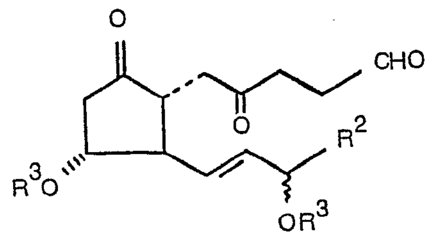

XV

in which $R^2$ and $R^3$ are as defined under formula I,

e) reacting the diketoaldehyde of the formula XV obtained with the ylide of the formula VI

$$(R^4)_3P = CH - COOCH_3 \qquad VI$$

in which the radicals $R^4$, which are identical or different, denote linear $C_1$—$C_4$ alkyl or phenyl to give a compound of the formula XVI

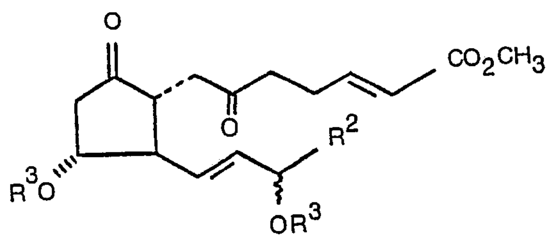

XVI

in which $R^2$ and $R^3$ are as defined under formula I, or

$f_1$) in the compound of the formula XV detaching the protective groups by acid hydrolysis, whereupon an aldehyde of the formula IX is obtained

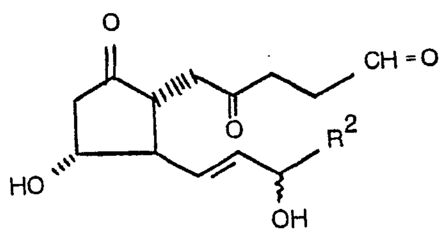

IX

in which $R^2$ is as defined under formula I, and

$f_2$) reacting the aldehyde of the formula IX with the ylide of the formula VI

$$(R^4)_3P = CH - COOCH_3 \qquad VI$$

in which the radicals $R^4$, which are identical or different, denote linear $C_1$—$C_4$ alkyl or phenyl to give a compound of the formula X

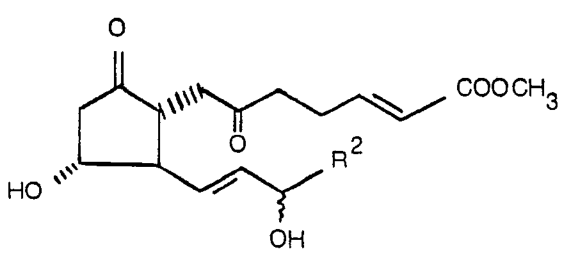

X

in which $R^2$ is as defined under formula I, or

g) in a compound of the formula

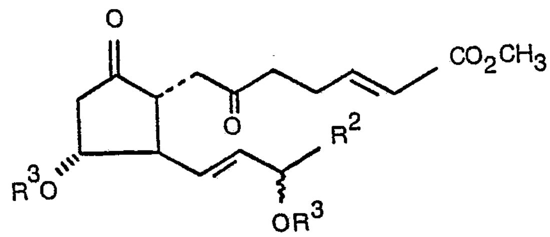

XVI

in which $R^2$ and $R^3$ are as defined under formula I optionally detaching the protective groups $R^3$ by acid hydrolysis, whereupon a compound of the formula X is obtained;

h) optionally reducing a compound of the formula X with a complex metal hydride to give a compound of the formula VII,

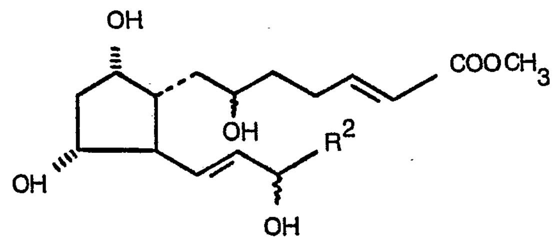

VII

i) optionally converting a compound of the formula VII, X or XVI by hydrolysis into a compound of the formula I in which $R^1$ denotes hydrogen and $X^1$, $Y^1$, $X^2$, $Y^2$, $R^2$ and $R^3$ are as defined under formula I;

k) optionally reducing a compound of the formula I, in which $R^1$ and $R^3$ denote hydrogen, $R^2$ is as defined under formula I and $X^1$ and $Y^1$ as well as $X^2$ and $Y^2$ together denote oxygen with a complex metal hydride to give a compound of the formula I in which $X^1$ and $Y^1$ as well as $X^2$ and $Y^2$ are different from each other and denote hydrogen or hydroxy;

l) optionally esterifying a compound of the formula I in which $X^1$, $X^2$, $Y^1$, $Y^2$, $R^2$ and $R^3$ are as defined under formula I and $R^1$ denotes hydrogen to give a compound of the formula I in which $X^1$, $X^2$, $Y^1$, $Y^2$, $R^2$ and $R^3$ are as defined under formula I and $R^1$ is not hydrogen, and

m) optionally converting a compound of the formula I in which $X^1$, $X^2$, $Y^1$, $Y^2$, $R^2$ and $R^3$ are as defined under formula I and $R^1$ denotes hydrogen into a physiologically acceptable metal or amine salt.

3. A process for the manufacture of a pharmaceutical composition, which comprises bringing a compound of the formula I as claimed in Claim 1, optionally in admixture or conjunction with the pharmaceutically suitable carriers and/or stabilizers, into a therapeutically suitable dosage unit form.

**Revendications pour les Etats contractants: BE CH DE FR GB IT NL SE**

1. Composés de formule I

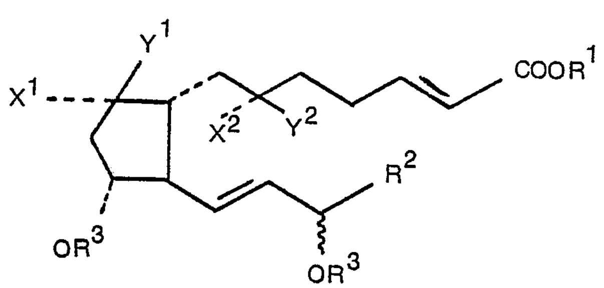

I

dans laquelle
$X^1$, $Y^1$ sont différents l'un de l'autre et représentent H ou OH,
$X^2$, $Y^2$ sont différents l'un de l'autre et représentent H ou OH, ou
$X^1$ et $Y^1$ ensemble ainsi que $X^2$ et $Y^2$ ensemble représentent l'oxygène dans chaque cas,

$R^1$ représente: a) l'hydrogène ou un radical hydrocarboné aliphatique ou cycloaliphatique, à chaîne droite, ou ramifiée, saturé ou insaturé, ayant jusqu'à 10 atomes de carbone ou

b) un ion métallique physiologiquement acceptable ou $NH_4^+$ ou un ion ammonium dérivant d'une amine primaire, secondaire ou tertiaire,

$R^2$ représente un radical alcoyle à chaîne droite ou ramifiée, ayant de 1 à 7 atomes de carbone, dans lequel un groupe $CH_2$ non terminal est remplacé par un atome d'oxygène, ou qui est substitué

a) par un radical $\alpha$- ou $\beta$-thiényl ou -furyle, qui de son côté peut être substitué de 1 à 3 fois sur le noyau par un halogène, un groupe trifluorométhyle et/ou alcoyle ou alcoxy ayant dans chaque cas de 1 à 6 atomes de carbone, ou

b) par un radical $\alpha$- ou $\beta$-thiényloxy ou par un radical cycloalcoxy ayant de 3 à 7 atomes de carbone, les radicaux mentionnés pouvant de leur côté être substitués de 1 à 3 fois sur le noyau par un halogène, un groupe trifluorométhyle et/ou alcoyle ou alcoxy ayant dans chaque cas de 1 à 6 atomes de carbone, et

$R^3$ représente l'hydrogène ou un groupe protecteur facilement éliminable.

2. Composés de formule 1 selon la revendication 1, caractérisés en ce que $X^1$ et $Y^1$ ensemble, ainsi que $X^2$ et $Y^2$ ensemble représentant dans chaque cas l'oxygène et $R^3$ représente l'hydrogène.

3. Procédé pour la préparation de composés de formula I

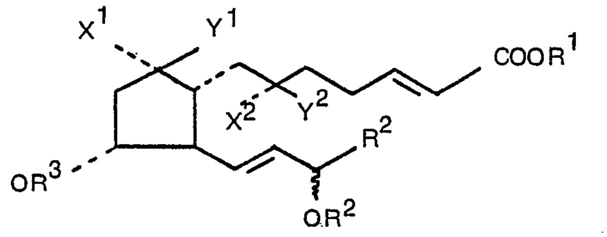

I

dans laquelle $R^1$, $R^2$, $R^3$, $X^1$, $X^2$ et $Y^2$ ont les significations indiquées pour la formule I dans la revendications 1, caractérisé en ce qu'on fait réagir un composé de formule XV

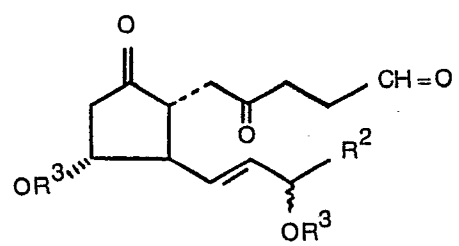

XV

dans laquelle $R^2$ et $R^3$ ont les significations indiquées pour la formule I, avec l'ylide de formule VI

$$(R^4)_3P = CH - COOCH_3 \qquad (VI)$$

dans laquelle les radicaux $R^4$ sont identiques ou différents et représentent un groupe alcoyle en $C_1$—$C_4$ â chaîne droite ou un groupe phényle, pour obtenir un ester de formule I, dans lequel $R^1$ représente le groupe méthyle; éventuellement dans l'ester de formule I obtenu, dans lequel $R^1$ représente le groupe méthyle et $R^3$ représente des groupes protecteurs facilement éliminables, on élimine les groupes protecteurs par une hydrolyse acide; éventuellement on convertit l'ester de formule I, dans lequel $R^1$ représente le groupe méthyle, par hydrolyse, en un composé de formule I, dans lequel $R^1$ représente l'hydrogène; éventuellement on réduit un composé de formule I, dans lequel $R^1$, $R^2$ et $R^3$ ont les significations indiquées pour le formule I dans le revendication 1, et $X^1$ et $Y^1$ ensemble ainsi que $X^2$ et $Y^2$ ensemble représentent dans chaque cas l'oxygène, en un composé de formule I, dans lequel $X^1$ et $Y^1$ sont différents et représentent H ou OH, de même que $X^2$ et $Y^2$ sont différents et représentent H ou OH; éventuellement on estérifie un composé de formule I, dans lequel $X^1$, $X^2$, $Y^1$, $Y^2$, $R^2$ et $R^3$ ont les significations indiquées pour la formule I dans la revendication 1 et $R^1$ représente l'hydrogène, en un composé de formule I, dans lequel $X^1$, $X^2$, $Y^1$, $Y^2$, $R^2$ et $R^3$ ont les significations indiquées pour la formule I dans la revendication 1, mais où $R^1$ n'est pas l'hydrogène; et éventuellement on convertit un composé de formule I, dans lequel $X^1$, $X^2$, $Y^1$, $Y^2$, $R^2$ et $R^3$ ont les significations indiquées pour la formule I dans la revendication 1 et $R^1$ représente l'hydrogène, en un sel métallique ou en un sel d'amine physiologiquement acceptable.

4. Procédé pour la préparation de composés de formule I

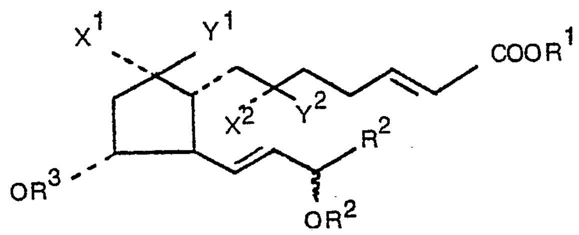

I

dans laquelle $X^1$, $Y^1$, $X^2$, $Y^2$, $R^1$, $R^2$ et $R^3$ ont les significations indiquées pour la formule I dans la revendication 1, caractérisé en ce que

a) on fait réagir un lactol de formule II

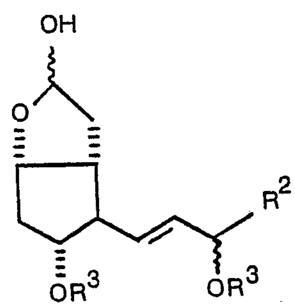

II

dans laquelle $R^2$ et $R^3$ ont les significations indiquées pour la formule I avec un composé de Grignard de formule XI

$$HalMg—CH_2—CH_2—CH=CH_2 \quad XI$$

dans laquelle Hal représente le chlore, le brome ou l'iode, pour obtenir un alcool de formule XII

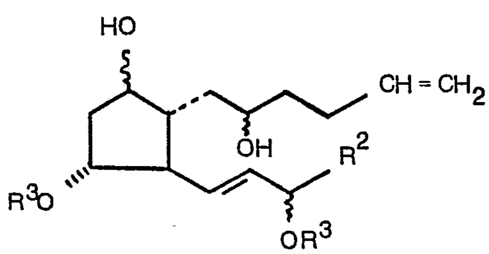

XII

dans laquelle $R^2$ et $R^3$ ont les significations indiquées pour la formule I,

b) on oxyde l'alcool de formule XII obtenu, obtenant ainsi une cétone de formule XIII

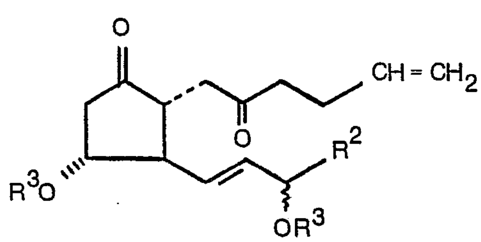

XIII

dans laquelle $R^2$ et $R^3$ ont les significations indiquées pour la formule I,

c) dans la dicétone de formule XIII obtenue, on oxyde sélectivement la double liaison terminale, obtenant ainsi un diol de formule XIV

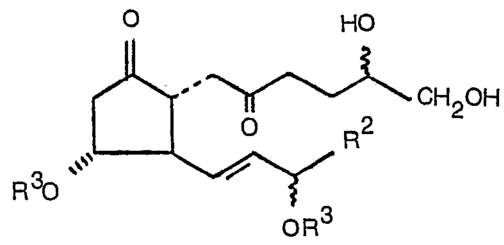

XIV

dans laquelle $R^2$ et $R^3$ ont les significations indiquées pour la formule I,

d) on oxyde le diol de formule XIV obtenu en un dicétoaldéhyde de formule XV

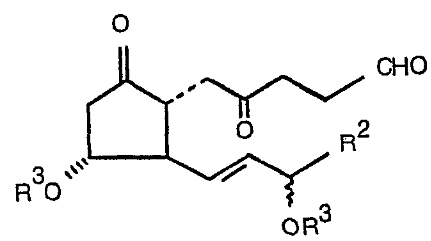

XV

dans laquelle $R^2$ et $R^3$ ont les significations indiquées pour la formule I,

e) on fait réagir le dicétoaldéhyde de formule XV obtenu avec l'ylide de formule VI

$$(R^4)_3P = CH - COOCH_3 \qquad VI$$

où les radicaux $R^4$ sont identiques ou différents et représentent un groupe alcoyle en $C_1$—$C_4$ à chaîne droite ou un groupe phényle, pour obtenir un composé de formule XVI

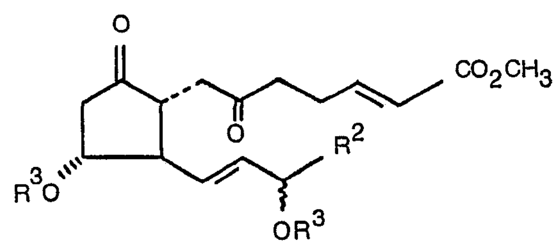

XVI

dans laquelle $R^2$ et $R^3$ ont les significations indiquées pour la formule I, ou

$f_1$) dans le composé de formule XV, on élimine les groupes protecteurs par hydrolyse acide, obtenant ainsi un aldéhyde de formule IX

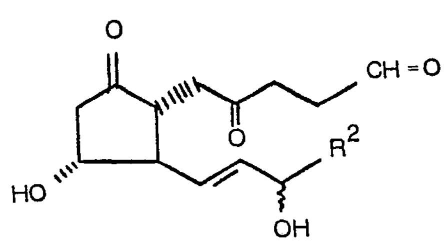

IX

dans laquelle $R^2$ a la signification indiquée dans la formule I, et

$f_2$) onfait réagir l'aldéhyde de formule IX avec l'ylide de formule VI

$$(R^4)_3P = CH - COOCH_3 \qquad VI$$

où les radicaux $R^4$ peuvent être identiques ou différents et représentent un groupe alcoyle en $C_1$—$C_4$ à chaîne droite ou un groupe phényle pour obtenir un composé de formule X

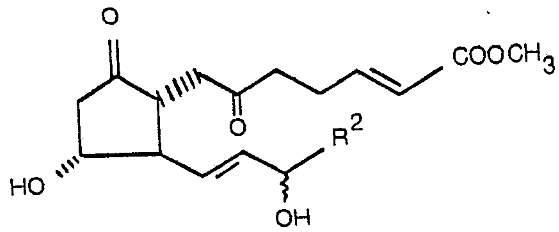

X

dans laquelle $R^2$ a la signification indiquée dans la formule I, ou

g) si on le désire, dans un composé de formule XVI

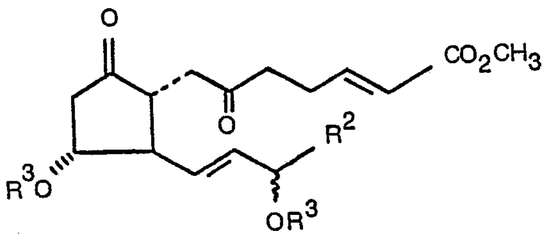

XVI

dans laquelle $R^2$ et $R^3$ ont les significations indiquées pour la formule I, on élimine les groupes protecteurs $R^3$ par hydrolyse acide, obtenant ainsi un composé de formule X, et

h) si on le désire, on réduit un composé de formule X avec un hydrure métallique complexe pour obtenir un composé de formule VII

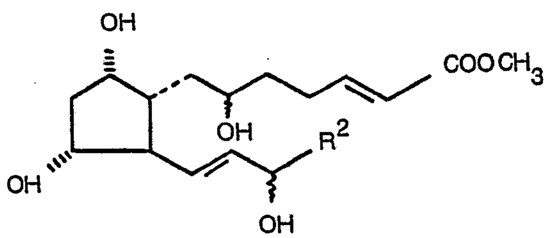

VII

i) si on le désire, on convertit un composé de formule VII, X ou XVI, par hydrolyse, en un composé de formule I dans lequel $R^1$ représente l'hydrogène et $X^1$, $Y^1$, $X^2$, $Y^2$, $R^2$ et $R^3$ ont les significations indiquées pour la formule I,

k) si on le désire, on réduit un composé de formule I, dans lequel $R^1$ et $R^3$ représentent l'hydrogène, $R^2$ a la signification indiquée pour la formule I et $X^1$ et $Y^1$ ainsi que $X^2$ et $Y^2$ représentent ensemble dans chaque cas l'oxygène avec un hydrure métallique complexe en un composé de formule I, dans lequel $X^1$ et $Y^1$ ainsi que $X^2$ et $Y^2$ sont différents les uns des autres et représentent l'hydrogène ou un groupe hydroxy,

l) si on le désire, on estérifie un composé de formule I, dans lequel $X^1$, $X^2$, $Y^1$, $Y^2$, $R^2$ et $R^3$ ont les significations indiquées pour la formule I et $R^1$ représente l'hydrogène, en un composé de formule I, dans lequel $X^1$, $X^2$, $Y^1$, $Y^2$, $R^2$ et $R^3$ ont les significations indiquées pour la formule I, mais où $R^1$ ne représente pas l'hydrogène, et

m) si on le désire, on convertit un composé de formule I, dans lequel $X^1$, $X^2$, $Y^1$, $Y^2$, $R^2$ et $R^3$ ont les significations indiquées pour la formule I et $R^1$ représente l'hydrogène, en un sel métallique ou en un sel d'amine physiologiquement acceptable.

5. Médicament, caractérisé par une teneur en un composé de formule I selon la revendication 1.

**Revendications pour l'Etat contractant: AT**

1. Procédé pour la préparation de composés de formule 1

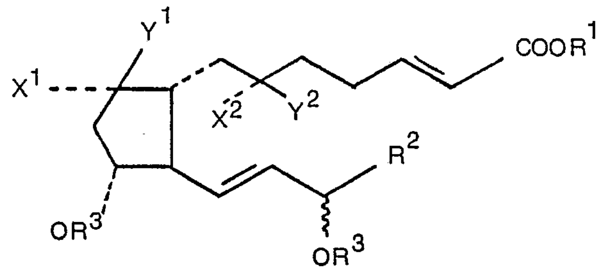

I

dans laquelle:

$X^1$, $Y^1$ sont différents l'un de l'autre et représentent H ou OH,

$X^2$, $Y^2$ sont différents l'un de l'autre et représentent H ou OH, ou

$X^1$ et $Y^1$ ensemble, ainsi que $X^2$ et $Y^2$ ensemble représentent dans chaque cas l'oxygène,

$R^1$ représente:

a) l'hydrogène on un radical hydrocarburé aliphatique ou cycloaliphatique, à chaîne droite, ou ramifiée, saturé on insaturé, ayant jusqu'à 10 atomes de carbone ou

b) un ion métallique physiologiquement acceptable ou $NH_4^+$ ou un ion ammonium dérivant d'une amine primaire, secondaire ou tertiaire,

$R^2$ représente un radical alcoyle à chaîne droite ou ramifiée, ayant de 1 à 7 atomes de carbone, dans lequel un groupe $CH_2$ non-terminal est remplacé par un atome d'oxygène, ou qui est substitué

a) par un radical $\alpha$- ou $\beta$-thiényle ou -furyle, qui de son côté peut être substitué de 1 à 3 fois sur le noyau par un halogène, un groupe trifluorométhyle, et/ou alcoyle ou alcoxy ayant de 1 à 6 atomes de carbone dans chaque cas ou

b) par un radical $\alpha$- ou $\beta$-thiényloxy ou un radical cycloalcoxy ayant de 3 à 7 atomes de carbone, les radicaux mentionnés pouvant de leur côté être substitués de 1 à 3 fois sur le noyau par un halogène, un groupe trifluorométhyle et/ou alcoyle ou alcoxy ayant dans chaque cas de 1 à 6 atomes de carbone, et

$R^3$ représente l'hydrogène ou un groupe protecteur facilement éliminable,

ce procédé étant caractérisé en ce qu'on fait réagir un composé de formule XV

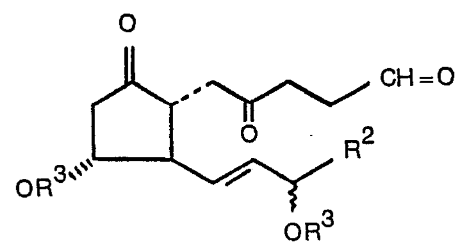

XV

dans laquelle $R^2$ et $R^3$ ont les significations indiquées pour la formule 1, avec un ylide de formule VI

$$(R^4)_3P = CH - COOCH_3 \qquad VI$$

dans laquelle les radicaux $R^4$ sont identiques ou différents et représentent un groupe alcoyle en $C_1$—$C_4$ à chaîne droite ou un groupe phényle, pour obtenir un ester de formule 1, dans lequel $R^1$ représente le groupe méthyle; éventuellement, dans l'ester de formule 1 obtenu, dans lequel $R^1$ représente le groupe méthyle et $R^3$ des groupes protecteurs facilement éliminables, on élimine les groupes protecteurs par hydrolyse acide; éventuellement, on convertit par hydrolyse l'ester de formule 1, dans lequel $R^1$ représente le groupe méthyle, en un composé de formule 1, dans lequel $R^1$ représente l'hydrogène; éventuellement, on réduit un composé de formule 1, dans lequel $R^1$, $R^2$ et $R^3$ ont les significations indiquées ci-dessus pour la formule 1 et $X^1$ et $Y^1$ ensemble, ainsi que $X^2$ et $Y^2$ ensemble représentent dans chaque cas l'oxygène, en un composé de formule 1, dans lequel $X^1$ et $Y^1$ sont différents et représentent H ou OH, de même que $X^2$ et $Y^2$ sont différents et représentent Hou OH; éventuellement, on estérifie un composé de formule 1, dans lequel $X^1$, $X^2$, $Y^1$, $Y^2$, $R^2$ et $R^3$ ont les significations indiquées ci-dessus pour la formule 1 et $R^1$ représente l'hydrogène, en un composé de formule 1, dans lequel $X^1$, $X^2$, $Y^1$, $Y^2$, $R^2$ et $R^3$ ont les significations indiquées ci-dessus pour la formule 1, mais où $R^1$ ne représente par l'hydrogène; et éventuellement, on convertit un composé de formule 1, dans lequel $X^1$, $X^2$, $Y^1$, $Y^2$, $R^2$

et $R^3$ ont les significations indiquées ci-dessus pour la formule 1 et $R^1$ représente l'hydrogène, en un sel métallique, ou en un sel d'amine physiologiquement acceptable.

2. Procédé pour la préparation de composés de formule 1

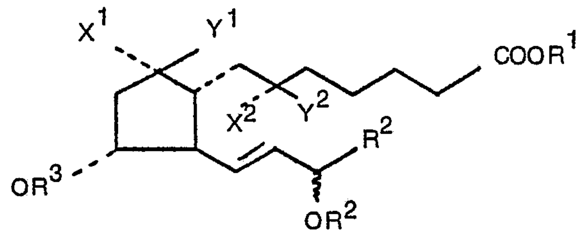

I

dans laquelle $X^1$ $Y^1$, $X^2$, $Y^2$, $R^1$, $R^2$ et $R^3$ ont les significations indiquées pour la formule 1 dans la revendications 1, caractérisé en ce que:

a) on fair réagir un lactol de formule II

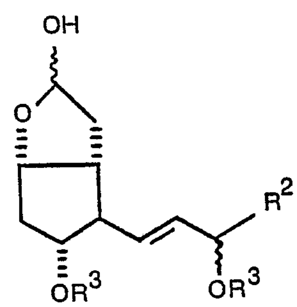

II

dans laquelle $R^2$ ou $R^3$ ont les significations indiquées pour la formule I, avec un composé de Grignard de formule XI

$$HalMg{-}CH_2{-}CH_2{-}CH{=}CH_2 \qquad XI$$

dans laquelle Hal représente le chlore, le brome ou l'iode, pour obtenir un alcool de formule XII

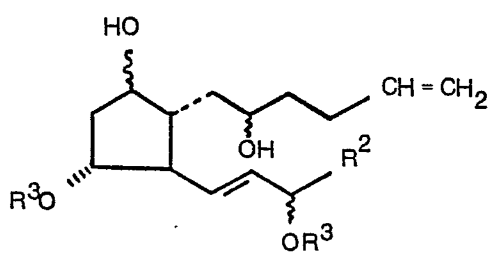

XII

dans laquelle $R^2$ et $R^3$ ont les significations indiquées pour la formule I,

b) on oxyde l'alcool de formule XII obtenu, obtenant aussi une cétone de formule XIII

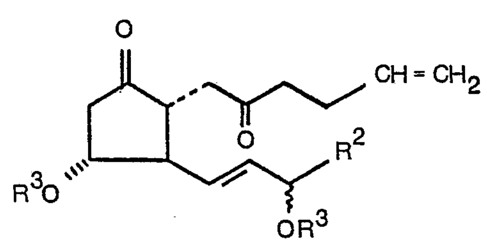

XIII

dans laquelle $R^2$ et $R^3$ on les significations indiquées pour la formule I,

c) dans la dicétone de formule XIII obtenue, on oxyde sélectivement la double liaison terminale, obtenant aussi un diol de formule XIV

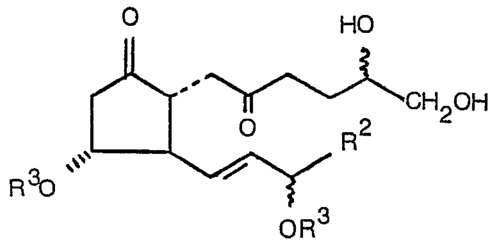

XIV

dans laquelle $R^2$ et $R^3$ ont les significations indiquées pour la formule I,

d) on oxyde le diol de formule XIV obtenu en un dicétoaldéhyde de formule XV

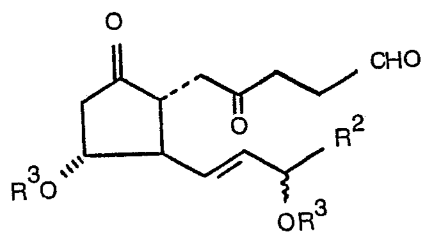

XV

dans laquelle $R^2$ et $R^3$ ont les significations indiquées dans la formule I,

e) on fait réagir le dicétoaldéhyde de formule XV obtenu avec l'ylide de formule VI

$$(R^4)_3P = CH - COOCH_3 \qquad VI$$

dans laquelle les radicaux $R^4$ sont identiques ou différents et représentent un groupe alcoyle en $C_1$—$C_4$ à chaîne droite ou un groupe phényle, pour obtenir un composé de formule XVI

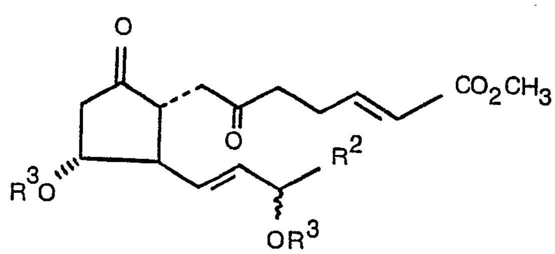

XVI

dans laquelle $R^2$ et $R^3$ ont les significations indiquées pour la formule I ou

$f_1$) dans le composé de formule XV, on élimine les groupes protecteurs par hydrolyse acide, obtenant aussi un aldéhyde de formule IX

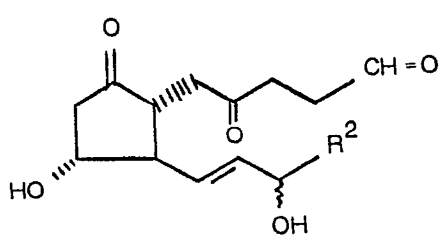

IX

dans laquelle $R^2$ a la signification indiquée dans la formule I, et

$f_2$) on fait réagir l'aldéhyde de formule IX avec l'ylide de formule VI

$$(R^4)_3P = CH - COOCH_3 \qquad VI$$

dans laquelle les radicaux $R^4$ peuvent être identiques on différents et représentent un groupe alcoyle en $C_1$—$C_4$ à chaîne droite ou un groupe phényle, pour obtenir un composé de formule X

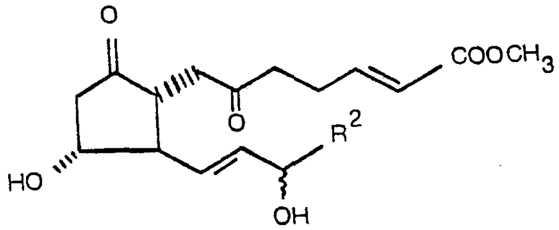

X

dans laquelle $R^2$ a la signification indiquée dans la formule I, ou

g) si on le désire, dans un composé de formule XVI

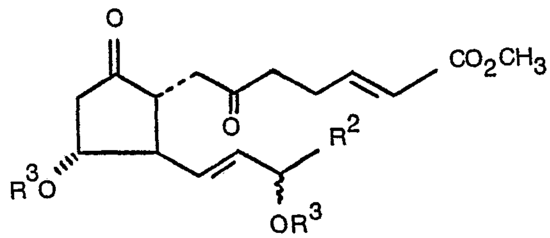

XVI

dans laquelle $R^2$ et $R^3$ ont les significations indiquées pour la formule I, on élimine les groupes protecteurs $R^3$ par hydrolyse acide, obtenant aussi un composé de formule X, et

h) si on le désire, on réduit un composé de formule X avec un hydrure métallique complexe en un composé de formule VII

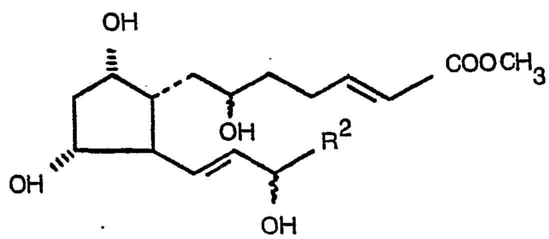

VII

i) si on le désire, on convertit un composé de formule VII, X ou XVI par hydrolyse en un composé de formule I, dans lequel $R^1$ représente l'hydrogène et $X^1$, $Y^1$, $X^2$, $Y^2$, $R^2$ et $R^3$ ont les significations indiquées pour la formule I,

k) si on le désire, on réduit un composé de formule 1, dans lequel $R^1$ et $R^3$ représentent l'hydrogène, $R^2$ a la signification indiquée pour la formule I, et $X^1$ et $Y^1$ ainsi que $X^2$ et $Y^2$ dans chaque cas ensemble représentent l'oxygène, avec un hydure métallique complexe en un composé de formule I, dans lequel $X^1$ et $Y^1$, ainsi que $X^2$ et $Y^2$ sont différents les uns des autres dans chaque cas et représentent l'hydrogène ou un groupe hydroxy,

l) si on le désire, on estérifie un composé de formule I, dans lequel $X^1$, $X^2$, $Y^1$, $Y^2$, $R^2$ et $R^3$ ont les significations indiquées pour la formule I, et $R^1$ représente l'hydrogène, en un composé de formule I, dans lequel $X^1$, $X^2$, $Y^1$, $Y^2$, $R^2$ et $R^3$ ont les significations indiquées pour la formule I, mais où $R^1$ ne représente par l'hydrogène, et

m) si on le désire, on convertit un composé de formule I, dans lequel $X^1$, $X^2$, $Y^1$, $Y^2$, $R^2$ et $R^3$ ont les significations indiquées pour la formule I, et $R^1$ représente l'hydrogène, en un sel métallique ou en un sel d'amine physiologiquement acceptable.

3. Procédé pour la préparation de médicaments, caractérisé en ce qu'on met un composé de formule I selon la revendication 1, éventuellement avec des véhicules et/ou des stabilisants pharmaceutiquement usuels, sous une forme d'administration thérapeutiquement appropriée.